# EUROPEAN PATENT SPECIFICATION

(11) **EP 2 626 417 B1**
(45) Date of publication and mention of the grant of the patent: **02.09.2015**
(21) Application number: 13164303.3
(22) Date of filing: 22.03.2007
(51) Int. Cl.: C12N 5/0775, A61K 35/12, C12N 5/073

(54) **Methods for cell expansion and uses of cells and conditioned media produced thereby for therapy**
Verfahren zur Zellexpansion sowie Verwendungen von damit produzierten Zellen und konditionierten Medien für die Therapie
Procédés de développement cellulaire et utilisations thérapeutiques des cellules et des milieux conditionnés produits de cette maniere

(30) Priority: 23.03.2006 US 784769 P; 26.09.2006 US 847088 P
(43) Date of publication of application: 14.08.2013
(62) Divisional of application: 11170055.5
(73) Proprietor: Pluristem Ltd., 31905 Haifa (IL)
(72) Inventor: Meretzki, Shai, 34990 Haifa (IL); Aberman, Zami, 40600 Tel-Mond (IL); Burger, Ora, 32882 Haifa (IL)
(74) Representative: Friedrich, Rainer

(56) References cited:
- WO-A1-00/46349
- LI CHANG DONG ET AL: "Mesenchymal stem cells derived from human placenta suppress allogeneic umbilical cord blood lymphocyte proliferation.", CELL RESEARCH JUL 2005, vol. 15, no. 7, July 2005 (2005-07), pages 539-547, XP009080356, ISSN: 1001-0602
- PUISSANT BÉNÉDICTE ET AL: "Immunomodulatory effect of human adipose tissue-derived adult stem cells: comparison with bone marrow mesenchymal stem cells.", BRITISH JOURNAL OF HAEMATOLOGY APR 2005, vol. 129, no. 1, April 2005 (2005-04), pages 118-129, XP2377924, ISSN: 0007-1048
- LAZARUS H M ET AL: "Cotransplantation of HLA-Identical Sibling Culture-Expanded Mesenchymal Stem Cells and Hematopoietic Stem Cells in Hematologic Malignancy Patients", BIOLOGY OF BLOOD AND MARROW TRANSPLANTATION, KLUGE CARDEN JENNINGS PUBLISHING, CHARLOTTESVILLE, VA, US, vol. 11, no. 5, 1 May 2005 (2005-05-01), pages 389-398, XP025312128, ISSN: 1083-8791, DOI: 10.1016/J.BBMT.2005.02.001 [retrieved on 2005-05-01]
- BURGER O ET AL: "Mesenchymal stromal cells, grown as high-density 3D culture, promote engraftment of human umbilical cord blood (hUCB) derived CD34+cells in NOD-SCID mice", EXPERIMENTAL HEMATOLOGY; 35TH ANNUAL MEETING OF THE INTERNATIONAL-SOCIETY-FOR-EXPERIMENTAL-HEM ATOLOGY; MINNEAPOLIS, MN, USA; SEPTEMBER 27 -30, 2006, ELSEVIER INC, US, vol. 34, no. 9, Suppl. 1, 1 September 2006 (2006-09-01), page 38, XP002656152, ISSN: 0301-472X
- YAN LI ET AL: "Effects of three-dimensional scaffolds on cell organization and tissue development", BIOTECHNOLOGY AND BIOPROCESS ENGINEERING, vol. 6, no. 5, 1 October 2001 (2001-10-01) , pages 311-325, XP055004175, ISSN: 1226-8372, DOI: 10.1007/BF02932999
- ZHANG Y ET AL: "COMPARISON OF MESENCHYMAL STEM CELLS FROM HUMAN PLACENTA AND BONE MARROW", CHINESE MEDICAL JOURNAL / ZHONGHUA YIXUE ZAZHI YINGWEN BAN, CHINESE MEDICAL ASSOCIATION, BEIJING, CN, vol. 117, no. 6, 1 June 2004 (2004-06-01), pages 882-887, XP009042592, ISSN: 0366-6999
- MOSBEUX C ET AL: "Mesenchymal cells: Metalloproteinases and adhesion on microcarriers", 1 January 2006 (2006-01-01), ANIMAL CELL TECHNOLOGY: BASIC & APPLIED ASPECTS, SPRINGER, NL, PAGE(S) 1 - 7, XP008121294, ISBN: 978-1-4020-4312-3 * the whole document *
- Q F WU ET AL: "Cultivation of human mesenchymal stem cells on macroporous CultiSpher G microcarriers", ZHONGGUO SHI YAN XUE YE XUE ZA ZHI, vol. 11, no. 1, 1 January 2003 (2003-01-01), pages 15-21, XP055042840,
- YANEZ R M ET AL: "IN VITRO AND IN VIVO IMMUNOMODULATORY EFFECTS OF MESENCHYMAL STEM CELLS FROM ADIPOSE TISSUE", BLOOD, AMERICAN SOCIETY OF HEMATOLOGY, US, vol. 106, no. 11. PART 01, 13 December 2005 (2005-12-13), page 866A, XP009065433, ISSN: 0006-4971
- NISHIMOTO N ET AL: "Treatment of rheumatoid arthritis with humanized anti-interleukin-6 receptor antibody: a multicenter, double-blind, placebo-controlled trial", ARTHRITIS & RHEUMATISM, WILEY, US, vol. 50, no. 6, 1 June 2004 (2004-06-01), pages 1761-1769, XP002398186, ISSN: 0004-3591, DOI: 10.1002/ART.20303
- M L Patchen ET AL: "Administration of interleukin-6 stimulates multilineage hematopoiesis and accelerates recovery from radiation-induced hematopoietic depression", Blood, 1 February 1991 (1991-02-01), page 472, XP055121251, UNITED STATES Retrieved from the Internet: URL:http://www.ncbi.nlm.nih.gov/pubmed/199 1164
- Xuetao Cao ET AL: "ACCELERATED RECOVERY OF IRRADIATION-INDUCED BONE MARROW DEPRESSION BY FIBROBLAST-MEDIATED INTERLEUKIN 6 GENE THERAPY IN COMBINATION WITH BONE MARROW TRANSPLANTATION IN MICE", Transplantation, vol. 65, no. 3, 15 February 1998 (1998-02-15), pages 325-331, XP055121265, ISSN: 0041-1337
- NASEF AISHA ET AL: "Leukemia inhibitory factor: Role in human mesenchymal stem cells mediated immunosuppression.", CELLULAR IMMUNOLOGY 2008 MAY-JUN, vol. 253, no. 1-2, May 2008 (2008-05), pages 16-22, ISSN: 1090-2163

## Description

### FIELD AND BACKGROUND OF THE INVENTION

The present invention relates to methods of cell expansion, populations of cells produced thereby and uses of same. Specifically the present invention relates to methods of expanding adherent cells from placenta or adipose tissues (along all the PCT) and therapeutic uses of same, such as for hematopoietic stem cell transplantation.

In the developing medical world a growing need exists for adult stem cells in large amounts for the purpose of cell engraftment and tissue engineering. In addition, adult stem cell therapy is continuously developing for treating and curing various conditions such as hematopoietic disorders, heart disease, Parkinson's disease, Alzheimer's disease, stroke, burns, muscular dystrophy, autoimmune disorders, diabetes and arthritis.

Hematopoietic stem cells (HSCs) are precursor cells, which give rise to all the blood cell types of both the myeloid and lymphoid lineages. Engraftment and initiation of hematopoiesis by transplanted HSCs depend on those cells ability to home and proliferate within the recipient BM.

It is widely accepted that stem cells are intimately associated in vivo with discrete niches in the marrow, which provide molecular signals that collectively mediate their differentiation and self-renewal, via cell-cell contacts or short-range interactions. These niches are part of the "hematopoietic inductive microenvironment" (HIM), composed of marrow cells, i.e. macrophages, fibroblasts, adipocytes and endothelial cells. The Marrow cells maintain the functional integrity of the HIM by providing extra cellular matrix (ECM) proteins and basement membrane components that facilitate cell-cell contact. They also provide various soluble or resident cytokines needed for controlled hematopoietic cell differentiation and proliferation.

The interactions between the HSC and the stroma are required to preserve the viability of the HSCs and prevent their differentiation. Following HSCs transplantation, the transplanted HSCs must home into the bone marrow (BM) microenvironment and lodge in the appropriate niches before they proliferate and differentiate. During the homing process, the transplanted HSCs leave the bloodstream and transmigrate by following a gradient of chemokines across the endothelial cell barrier of the BM to reach the dedicated niches. The donor HSCs must then home into the hematopoietic niches where they encounter a more favorable microenvironment for HSC division, and where, a continuum, physical and chemical contacts can be established between the HSCs and the mesenchymal cells, the ECM and the secreted growth factors. All these processes involve a complex array of molecules, such as cytokines, chemokines, hormones, steroids, extra cellular matrix proteins, growth factors, cell-to-cell interaction proteins, adhesion proteins, and matrix proteins.

The total number of cells engrafted in the BM dedicated niches underlies the success of HSCs transplant. To achieve engraftment, donor HSCs that are transplanted into the blood circulation should home into the recipient's marrow where they generate functional hematopoiesis foci. The number of these foci is concluded as the product of total HSCs transfused multiplied by their engraftment efficiency.

One of the major problems involved with HSC transplantation is the low survival rate of these cells in the acceptor system. It is well documented that HSC transplanted intravenously are cleared from the circulation and visualized in the BM within minutes after their transfusion. Three to five hours after HSCs transplantation, no donor cells are detected in the peripheral blood of the recipients [Askenasy et al 2002 Transplanted hematopoietic cells seed in clusters in recipient bone marrow in vivo. Stem Cells. 20:301-10]. The vast majority of the transplanted cells are destroyed shortly after being transfused. Consequently, the colonization of the recipient's marrow is of low efficiency and only 1-5 % of the transfused cells are detected in the recipient BM 2-3 days post transplantation [Kerre et al 2001 2001 Both CD34+38+ and CD34+38- cells home specifically to the bone marrow of NOD/LtSZ scid/scid mice but show different kinetics in expansion. J Immunol. 167:3692-8; Jetmore et al 2002 2002 Homing efficiency, cell cycle kinetics, and survival of quiescent and cycling human CD34(+) cells transplanted into conditioned NOD/SCID recipients. Blood. 99:1585-93].

Mesenchymal Stromal Cells (MSCs) are a heterogeneous population of cells, capable of differentiating into different types of mesenchymal mature cells. The differentiation of these cells to reticular endothelial cells, fibroblasts, adipocytes, and osteogenic precursor cells, depend upon influences from various bioactive factors.

The use of MSCs for the support of HSC engraftment is known in the art. Several publications have demonstrated higher engraftment efficiencies of HSC when co-transplanted with mesenchymal stem cells [Gurevitch et al 1999 1999 Transplantation of allogeneic or xenogeneic bone marrow within the donor stromal microenvironment. Transplantation. 68:1362-8; Fan et al 2001 2001 Successful allogeneic bone marrow transplantation (BMT) by injection of bone marrow cells via portal vein: stromal cells as BMT-facilitating cells. [Stem Cells. 19:144-50]. It was also demonstrated that co-transplantation of human mesenchymal stem cells in a human-sheep engraftment model, resulted in the enhancement of long-term engraftment of human HSC chimeric BM in the animals [Almeida-Porada et al 2000] Co-transplantation of human stromal cell progenitors into preimmune fetal sheep results in early appearance of human donor cells in the circulation and boosts cell levels in bone marrow at later time points after transplantation [Blood. 95:3620-7]. It was found that simultaneous injection of HSC and mesenchymal stem cells accelerated hematopoiesis [Zhang et al 2004. Stem Cells 22:1256-62]. Recently, these finding were extended to a closer animal model - the Rhesus monkey. When haplo-identical HSC and mesenchymal stem cells were co-transplanted, facilitated HSC engraftment was demonstrated [Liu et al 2005 Zhonghua Xue Ye Xue Za Zhi. 26:385-8]. The use of mesenchymal stem cells to promote engraftment of HSC in human subjects was also recently reported [Koc ON, J Clin Oncol. 2000;18:307-316; Lazarus HM, Biol Blood Marrow Transplant. 2005 May;11(5):389-98].

Apparently the MSCs contribution to hematopoietic engraftment lies in the production of HSC supporting cytokines that help mediating and balancing the homing, self-renewal and commitment potentials of the transplanted HSCs, in rebuilding the damaged hematopoietic microenvironment needed for the homing and proliferation of the HSCs and in the inhibition of the donor derived T cells, which may cause Graft vs. Host Disease (GvHD), [Charbord P., and Moore, K., Ann. N.Y. Acad. Sci. 1044: 159-167 (2005); US patent nos. 6,010,696; 6555374]. For example, in a study by Maitra, [Maitra B, et al., Bone Marrow Transplant. 33(6):597-604. (2004)], human mesenchymal stem cells were found to support unrelated donor hematopoietic stem cells and suppressed T-cell activation in NOD-SCID mice model, showing that unrelated, human bone marrow-derived MSCs may improve the outcome of allogeneic transplantation.

One major obstacle in using MSCs is the difficulty of isolating large quantities of normally occurring populations of these cells, which is technically difficult and costly, due in part, to the limited quantity of cells. The most obvious source of MSCs is the bone marrow, but the significant discomfort involved in obtaining bone marrow aspirates and the risk of biopsy serve as drawbacks to these methods. The widely held belief that the human embryo and fetus constitute independent life makes the human embryo a problematic source of stem cells, adding a religious and ethical aspect to the already existing logistic difficulties.

Finding alternative sources for harvesting stem cells, has recently been attempted. Such alternative sources are for example adipose tissue, hair follicles, testicles, human olfactory mucosa, embryonic yolk sac, placenta, adolescent skin, and blood (e.g., umbilical cord blood and even menstrual blood). However, harvesting of stem cells from the alternative sources in adequate amounts for therapeutic and research purposes is still limited and generally laborious, involving, e.g., harvesting cells or tissues from a donor subject or patient, culturing and/or propagation of cells in vitro, dissection, etc.

The placenta is considered to be one of the most accessible sources of stem cells that does not involve any discomfort or ethical restraints. Placenta derived MSCs were found to have similar properties as BM derived MSC. They are plastic-adherent, express CD105, CD73 and CD90 membrane markers, and lack the expression of CD45, CD34, CD14, CD19 and HLA-DR surface molecules. However, unlike BM derived MSCs, placenta derived (PD)-MSCs treated with interferon- y very minimally upregulated HLA-DR. Moreover, PD-MSCs cells exhibit immunosuppressive properties that are enhanced in the presence of interferon-γ. (Chang CJ, Yen ML, Chen YC, Chien CC, Huang HI, Bai CH, Yen BL. Placenta-derived Multipotent Cells exhibit immunosuppressive properties that are enhanced in the presence of interferon-gamma. Stem Cells. 2006 Nov;24(11):2466-77.

In addition to MSC markers PD-MSCs exhibit unique ESC surface markers of SSEA-4, TRA-1-61, and TRA-1-80, that suggest that these may be very primitive cells. (Yen BL, Huang HI, Chien CC, Jui HY, Ko BS, Yao M, Shun CT, Yen ML, Lee MC, Chen YC. Isolation of multipotent cells from human term placenta. Stem Cells. 2005;23(1):3-9). Moreover, PD-MSCs (Fetal origin), but not BM derived MSC are positive for the intracellular human leukocyte antigen-G (HLA). ? (Chang CJ, Yen ML, Chen YC, Chien CC, Huang HI, Bai CH, Yen BL. Placenta-derived multipotent cells exhibit immunosuppressive properties that are enhanced in the presence of interferon-gamma. Stem Cells. 2006 Nov;24(11):2466-77.)

Studies have shown that the expansion potential of PD- MSCs was significantly higher than that of adult BM-derived MSCs (Yen BL, Huang HI, Chien CC, Jui HY, Ko BS, Yao M, Shun CT, Yen ML, Lee MC, Chen YC. Isolation of Multipotent cells from Human Term Placenta. Stem Cells. 2005;23(1):3-9; M.J.S. de Groot-Swings, Frans H.J. Claas, Willem E. Fibbe and Humphrey H.H. Pieternella S. in 't Anker, Sicco A. Scherjon, Carin Kleijburg-van der Keur, Godelieve. Placenta Isolation of Mesenchymal Stem Cells of Fetal or Maternal Origin from Human. Sem Cells, 2004;22;1338-1345.) In addition the placenta derived adherent cells can differentiate to osteoblasts, adipocytes and chondroblasts. Like BM derived MSCs, placenta derived MSCs were found to suppress umbilical cord blood (UCB) lymphocyte proliferation suggesting that combined transplantation of HSC and placenta derived (PD)-MSCs can reduce the potential graft-versus-host disease (GvHD) in recipients [Li CD, et al., Cell Res. Jul;15(7):539-47 (2005)], and can enhance hematopoietic support [ZhangYi et al., Chinese Medical Journal117(6): 882-887 (2004)]. The use of the placenta as a source for amniotic epithelial cells is taught for example in WO 00/73421, but obtaining these cells is still labor-intensive and the yield of the MSCs is very low.

Another way to solve the problem of the limited amount of MSCs is *ex-vivo* expansion of these cells using different culturing conditions [e.g. US Patent Nos. 6,326,198; 6030836; 6555374; 6,335,195; 6,338,942]. However, the drawback of such methods remains in the time-consuming, specific selection and isolation procedures they require, rendering these methods costly and fastidious.

Three dimensional (3D) culturing of cells was found in several studies to be more effective in yield [Ma T, et al., Biotechnology Progress. Biotechnol Prog 15:715-24 (1999); Yubing Xie, Tissue Engineering 7(5): 585-598 (2001)]. The Use of 3D culturing procedures which mimic the natural environment of the MSCs is based on seeding these cells in a perfusion bioreactor containing Polyactive foams [Wendt, D. et al., Biotechnol Bioeng 84: 205-214, (2003)] tubular poly-L-lactic acid (PLLA) porous scaffolds in a Radial-flow perfusion bioreactor [Kitagawa et al., Biotechnology and Bioengineering 93(5): 947-954 (2006)], and a plug flow bioreactor for the growth and expansion of hematopoietic stem cells (U.S. Patent No. 6,911,201).

A three-dimensional framework, which attaches stromal cells, was suggested in U.S. Pat. No. 6,022,743, and sponge collagen was suggested as a 3D matrix in Hosseinkhani, H et al., [Tissue Engineering 11(9-10): 1476-1488 (2005)]. However, the use of MSCs, grown in these conditions for supporting *in vivo* engraftment of HSCs following HSC transplantation has never been suggested in any of these studies. Also, time consuming optimization of various conditions e.g., perfusion conditions, or various isolation techniques for specific cell types were required.

The use of a perfused Post-partum placenta as a 3D reactor for culturing MSCs was suggested in US Pat. No. 7045148 and U.S. Pat. App. Nos. 20020123141 20030032179 and 2005011871. However, this procedure is limited for up to 24 hours after the placenta is isolated and involves perfusion, therefore mass growth of the cells and its maintenance for prolonged time periods is not possible. Lazarus (Biology of Blood and Marrow Transplantation 11 (2005), pp. 389-398) discloses that cotransplantation of HLA-identical sibling MSCs with an HLA-identical sibling HSC transplant is feasible.

There is thus a widely recognized need for, and it would be highly advantageous to have, novel methods of cell expansion and uses of cells and conditioned medium produced thereby for therapy and which are devoid of the above limitations.

### SUMMARY OF THE INVENTION

According to one aspect of the present invention there is provided a method of cell expansion, the method comprising culturing adherent cells from placenta or adipose tissue under three-dimensional culturing conditions, which support cell expansion.

According to another aspect of the present invention there is provided a method of producing a conditioned medium, the method comprising: culturing adherent cells from a placenta or adipose tissue in three dimensional culturing conditions which allow cell expansion; and collecting a conditioned medium of the expanded adherent cells, thereby producing the conditioned medium.

According to yet another aspect there is provided a population of cells generated according to the method as above.

According to still another aspect there is provided an isolated population of cells comprising adherent cells of placenta or adipose tissue, wherein the adherent cells secrete a higher level of at least one factor selected from the group consisting of SCF, IL-6, and Flt-3 than that secreted by adherent cells of placenta or adipose tissue grown in a 2D culture.

According to an additional aspect there is provided an isolated population of cells comprising adherent cells of placenta or adipose tissue, wherein the adherent cells express a higher level of at least one protein selected from

Li et al. (Cell Research 15(2005) pp. 539-547), Puissant et al. (British Journal of Haematology 129(2005), pp. 118-119) and Yanez et al. (Blood, 106(2005), p. 866A) all disclose the immunomodulatory activity of MSCs from placenta or adipose tissue.

the group consisting of H2A histone family (H2AF), Aldehyde dehydrogenase X (ALDH X), eukaryotic translation elongation factor 2 (EEEF2), reticulocalbin 3, EF-hand calcium binding domain (RCN2) and calponin 1 basic smooth muscle (CNN1) than that expressed by adherent cells of placenta or adipose tissue grown in a 2D culture.

According to yet an additional aspect there is provided an isolated population of cells comprising adherent cells of placenta or adipose tissue, wherein the adherent cells express a lower level of expression of at least one protein selected from the group consisting of heterogeneous nuclear ribonucleoprotein H1 (Hnrph1), CD44 antigen isoform 2 precursor, 3 phosphoadenosine 5 phosphosulfate synthase 2 isoform a (Papss2) and ribosomal protein L7a (rpL7a) than that expressed by adherent cells of placenta or adipose tissue grown in a 2D culture.

According to still an additional aspect there is provided an isolated population of cells comprising adherent cells of placenta or adipose tissue, wherein the adherent cells are characterized by a higher immunosuppressive activity than that of adherent cells of placenta or adipose tissue grown in a 2D culture.

According to further features in preferred embodiments of the invention described below the immunosuppressive activity comprises reduction in T cell proliferation.

According to further aspect there is provided a pharmaceutical composition comprising, as an active ingredient, the population of cells generated according to the method as above.

According to a further aspect there is provided a pharmaceutical composition comprising, as an active ingredient, the conditioned medium produced according to the method as above.

According to yet a further aspect there is provided a pharmaceutical composition comprising, as an active ingredient, the isolated population of cells according to above.

According to still a further aspect of the present invention there is provided a method of treating a condition which may benefit from stromal cell transplantation in a subject in need thereof, the method comprising administering to the subject a therapeutically effective amount of adherent cells of a tissue selected from the group consisting of placenta and adipose tissue obtained from a three dimensional culture, thereby treating the condition which may benefit from stem cell transplantation in the subject.

According to still a further aspect there is provided a method of treating a condition which may benefit from stromal cell transplantation in a subject in need thereof, the method comprising administering to the subject a therapeutically effective amount of a conditioned medium of adherent cells derived from a tissue selected from the group consisting of placenta and adipose tissue, thereby treating the condition which may benefit from stem cell transplantation in the subject.

According to still a further aspect there is provided a method of reducing an immune response in a subject in need thereof, the method comprising administering to the subject a therapeutically effective amount of the isolated population of cells of claims 3, 4, 5, 6 or 7, so as to reduce the immune response in the subject.

According to still further features in the described preferred embodiments the subject is treated with cell therapy.

According to still further features in the described preferred embodiments the method further comprises administering stem cells.

According to still further features in the described preferred embodiments the stem cells comprise hematopoietic stem cells.

According to still further features in the described preferred embodiments the cells are administered concomitantly with the conditioned medium or adherent cells.

According to still further features in the described preferred embodiments the cells are administered following administration of the conditioned medium or adherent cells.

According to still further features in the described preferred embodiments the adherent cells are obtained from a three dimensional culture.

According to still further features the adherent cells are obtained from a two dimensional culture.

According to still further features in the described preferred embodiments the condition that may be treated with the (conditioned medium of) adherent stromal cells is selected from the group consisting of autoimmune disorders, arthritis, Multiple Sclerosis, graft vs. host disease (GvHD), autoimmune encephalomyelitis (EAE), systemic lupus erythematosus (SLE), rheumatoid arthritis, systemic sclerosis, Sjorgen's syndrome, Myasthenia Gravis (MG), Guillain-Barré Syndrome (GBS), Hashimoto's Thyroiditis (HT), Graves's Disease, Insulin dependent Diabetes Melitus (IDDM) and Inflammatory Bowel Disease With the adherent stromal cells, also loss of blood and anemia can be treated.

According to still further features in the described preferred embodiments the three dimensional culture comprises a 3D bioreactor.

According to still further features in the described preferred embodiments the bioreactor is selected from the group consisting of a plug flow bioreactor, a continuous stirred tank bioreactor and a stationary-bed bioreactor.

According to still further features in the described preferred embodiments the culturing of the cells is effected under a continuous flow of a culture medium.

According to still further features in the described preferred embodiments the three dimensional culture comprises an adherent material selected from the group consisting of a polyester, a polyalkylene, a polyfluorochloroethylene, a polyvinyl chloride, a polystyrene, a polysulfone, a cellulose acetate, a glass fiber, a ceramic particle, a matrigel, an extracellular matrix component, a collagen, a poly L lactic acid and an inert metal fiber.

According to still further features in the described preferred embodiments the culturing is effected for at least 3 days.

According to still further features in the described preferred embodiments the culturing is effected for at least 3 days.

According to still further features in the described preferred embodiments the culturing is effected until the adherent cells reach at least 60 % confluence.

According to still further features in the described preferred embodiments the condition may benefit from the facilitation of hematopoietic stem cell engraftment.

According to still further features in the described preferred embodiments the adherent cells comprise a positive marker expression array selected from the group consisting of CD73, CD90, CD29 and CD105.

According to still further features in the described preferred embodiments the adherent cells comprise a negative marker expression array selected from the group consisting of CD45, CD80, HLA-DR, CD11b, CD14, CD19, CD34 and CD79.

According to still further features in the described preferred embodiments the adherent cells secrete a higher level of at least one factor selected from the group consisting of SCF, Flt-3 and IL-6 higher than that secreted by adherent cells from placenta or adipose tissue grown in a 2D culture.

According to still further features in the described preferred embodiments the adherent cells express a higher level of at least one protein selected from the group consisting of H2A histone family (H2AF), Aldehyde dehydrogenase X (ALDH X), eukaryotic translation elongation factor 2 (EEEF2), reticulocalbin 3, EF-hand calcium binding domain (RCN2) and calponin 1 basic smooth muscle (CNN1) than that secreted by adherent cells from placenta or adipose tissue grown in a 2D culture.

According to still further features in the described preferred embodiments the adherent cells express a lower level of expression of at least one protein selected from the group consisting of heterogeneous nuclear ribonucleoprotein H1 (Hnrph1), CD44 antigen isoform 2 precursor, 3 phosphoadenosine 5 phosphosulfate synthase 2 isoform a (Papss2) and ribosomal protein L7a (rpL7a) than that secreted by adherent cells from placenta or adipose tissue grown in a 2D culture.

According to still further features in the described preferred embodiments the adherent cells or medium are characterized by a higher immunosuppressive activity than that of adherent cells of placenta or adipose tissue grown in a 2D culture.

According to still further features in the described preferred embodiments the immunosuppressive activity comprises reduction in T cell proliferation.

According to still further features in the described preferred embodiments the cells comprise cells having a stromal stem cell phenotype.

According to still further features in the described preferred embodiments the stromal stem cell phenotype comprises T cell suppression activity.

According to still further features in the described preferred embodiments thstromal stem cell phenotype comprises hematopoietic stem cell support activity.

According to still further features in the described preferred embodiments the use of the population of cells described above is for manufacture of a medicament identified for transplantation.

The present invention successfully addresses the shortcomings of the presently known configurations by providing novel methods of cell expansion and uses of cells and conditioned medium produced thereby for therapy.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the patent specification, including definitions, will control. In addition, the materials, methods, and examples are illustrative only and not intended to be limiting.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

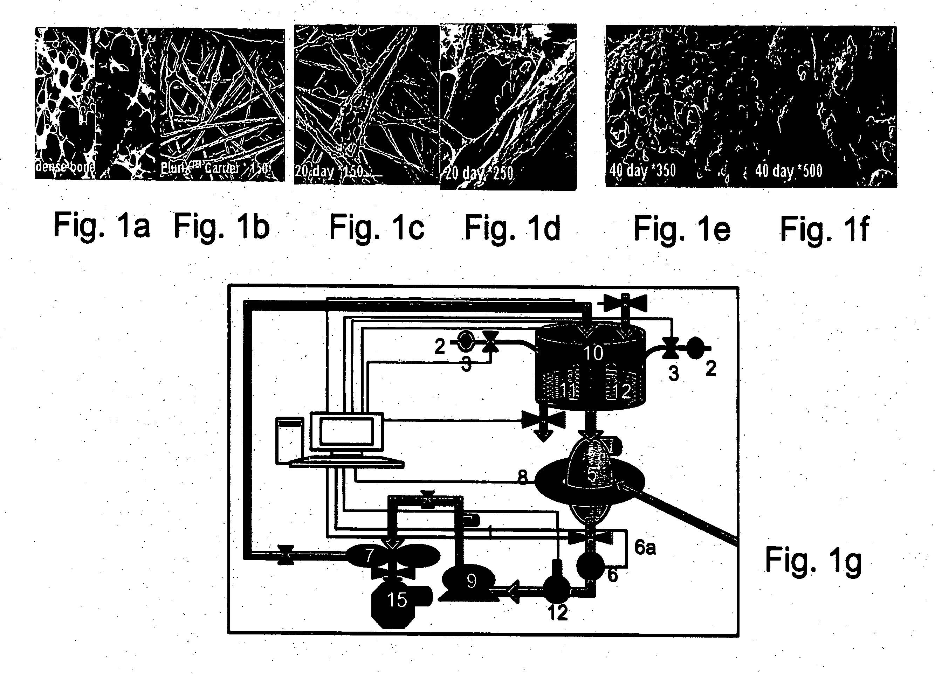

FIGs. 1a-g depicts the bone-like microenvironment created in the bioreactor system containing 3-D carriers. Figures 1a-b are electron micrographs depicting the comparison of natural bone (Figure 1a) and the structure of the PluriX™ 3D carrier 7 days after seeding Adherent Stromal Cells (3D-ASC), imitating the bone microenvironment (Figure 1b). Figures 1c-f are electron micrographs depicting the PluriX™ 3D matrix seeded with 3D-ASC, produced from bone marrow, 20 days (Figures 1c-d, magnified X 150 and 250 respectively) and 40 days (Figures le-f, magnified X 350 and 500 respectively) after seeding. Figure 1g is a diagram of the Plurix 3D plug flow bioreactor with separate parts defined by numbers: Culture medium reservoir (1), gas mixture supply (2), filter (3), injection point (4), column in which the 3D carriers are placed (5) flow monitor (6), flow valve (6a), separating container (7), cell growth analyzers (8); peristaltic pump (9), sampling point (10), dissolved O₂ measurement electrode (11), pH measurement electrode (12), control system (13), fresh growth media (14), used growth media (15).

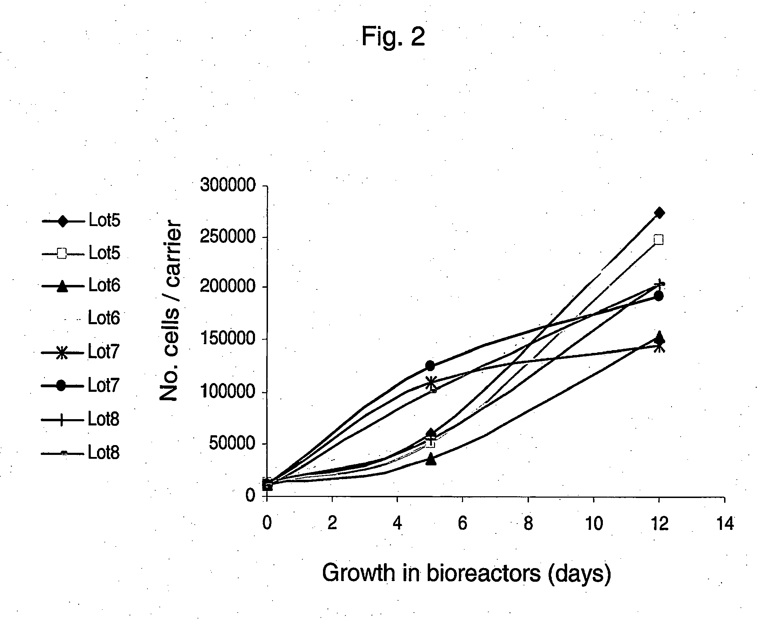

FIG. 2 is a graph depicting different production lots of adherent stromal cells (3D-ASC; Lots 5-8) originating from_placenta, grown in 3D growth conditions within the bioreactor systems. ASCs (2 X 10⁶) were seeded in the bioreactor at a density of 10000 - 15000 cells / a carrier. Following a 12 day culture 3D-ASCs reached a density of between 150,000-250,000 cells /carrier or 22.5-37.5 X 10⁶ in a bioreactor containing 150 carriers.

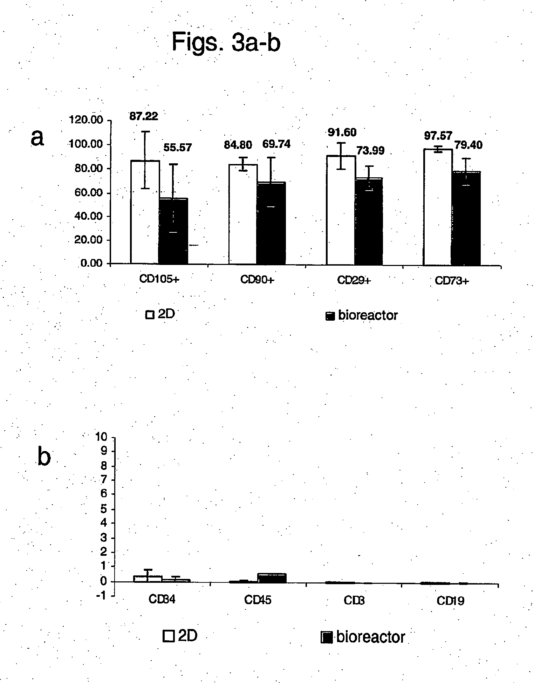

FIGs. 3a-b are bar graphs depicting difference in expression levels of expressed membrane markers in placenta derived 3D-ASC (dark purple) as compared to membrane markers in placenta cells cultured in conventional 2D culture conditions (light purple). Adherent cells were grown for 4-6 weeks in flasks (2D) or for 2-3 weeks in the bioreactor system, on polystyrene carriers (3D). Following harvesting from either flasks or carriers, cells were incubated and bound to a panel of monoclonal antibodies (MAb), which recognize membrane markers characteristic of MSCs (Figure 3a), or hematopoietic cells (Figure 3b). Note the significantly higher expression of MSC membrane markers in 2D cultured cells as shown for CD90, CD105, CD73 and CD29 membrane markers, compared to MSC membrane markers expressed in 3D-cultured adherent cells, especially CD105 which showed 56 % expression in 3D cultured cells vs. 87 % in the 2D cultured cells (Figure 3a). ASCs of both 2D and 3D cultures, did not express any hematopoietic membrane markers (Figure 3b).

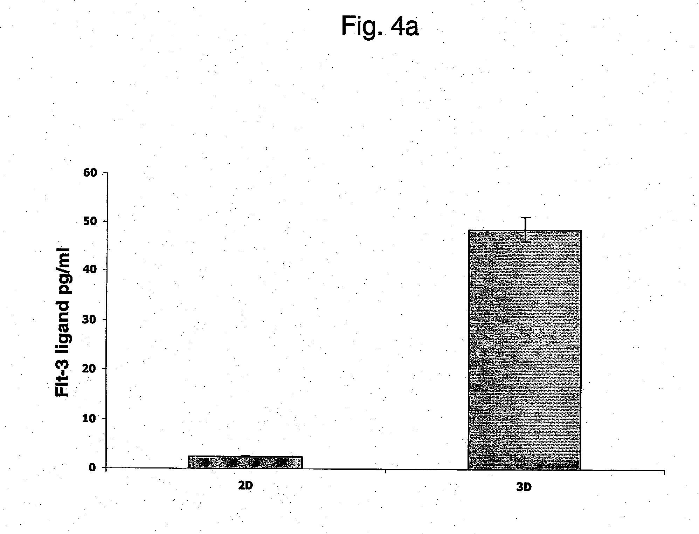

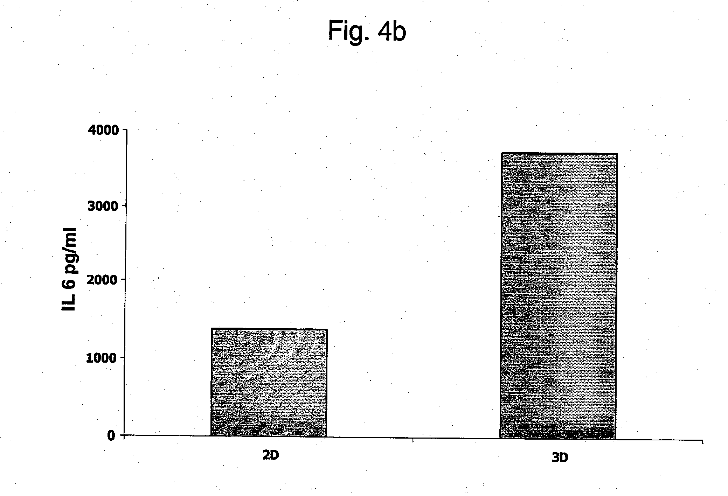

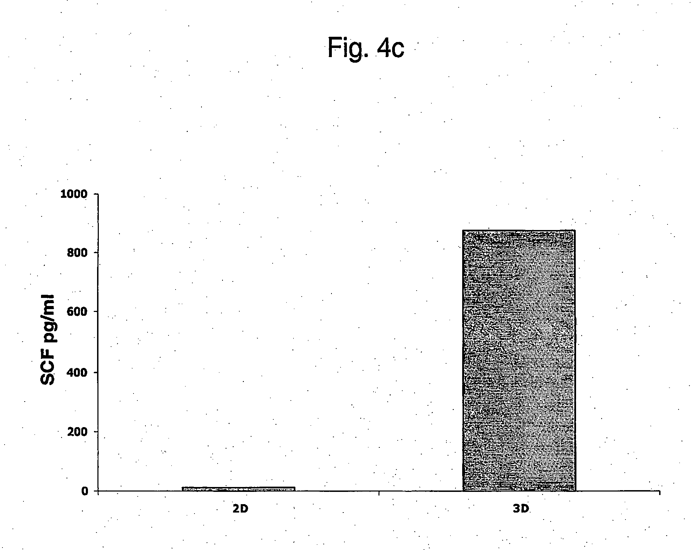

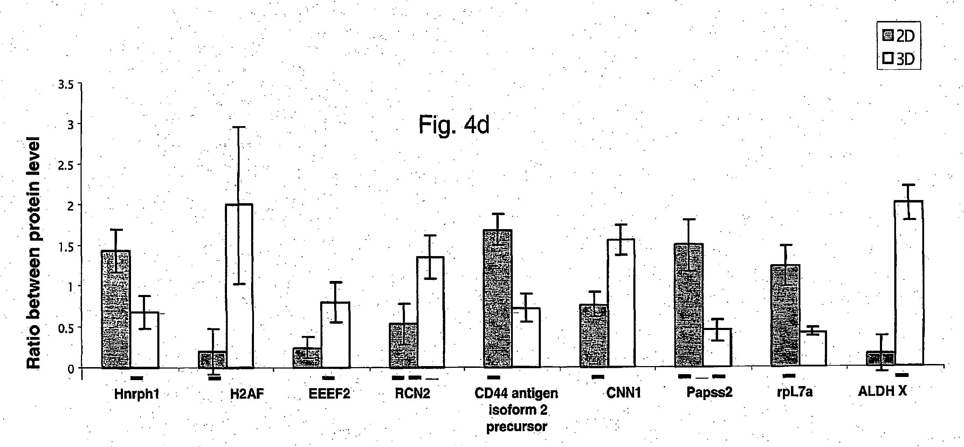

FIGs. 4a-d are bar graphs depicting a comparison of protein levels in ASCs produced from the placenta cultured under 2D and 3D Conditions or conditioned media of same. Figures 4a-c depict levels of Flt-3 ligand (Figure 4a), IL-6 (Figure 4b) and SCF (Figure 4c) in pg/ml, normalized for 1 X 10⁶ cells/ml, as analyzed by ELISA, in the conditioned media of 2D and 3D cultured ASCs. Results represent one of three independent experiments. Figure 4d shows the expression levels of different cellular proteins, as analyzed by mass spectrometry with iTRAQ reagents labeled protein samples compared therebetween. Protein samples were taken from ASCs grown under 2D (white bars) and 3D (grey bars) conditions. The figure represents one of two replica experiments. Note the difference in expression level of some of the proteins in cells and conditioned media of 2D and 3D culture conditions.

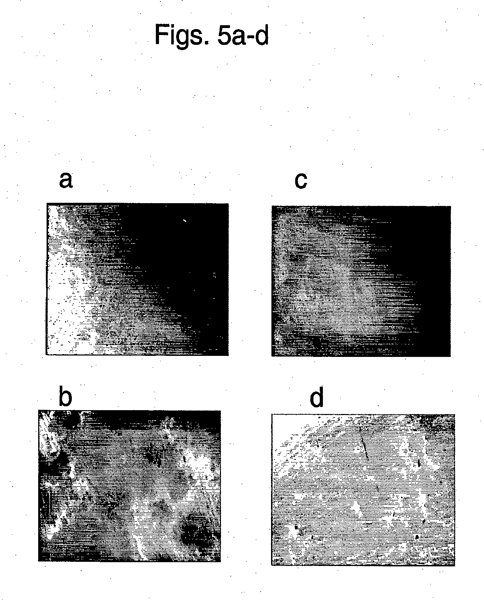

FIGs. 5a-d are micrographs depicting *in vitro* differentiation capability of placenta derived 3D-ASC to osteoblasts. Human placenta derived ASC were cultured in an osteogenic induction medium (DMEM containing 10 % FCS, 100 nM dexamethasone, 0.05 mM ascorbic acid 2-phosphate, 10 mM B-glycerophosphate) for a period of 3 weeks. Figures 5a-b show cells expressing calcified matrix, as indicated by Alizzarin Red S staining. Figures 5c-d show control cells, which were not treated with osteogenic induction medium and maintained a fibroblast like phenotype and demonstrating no mineralization.

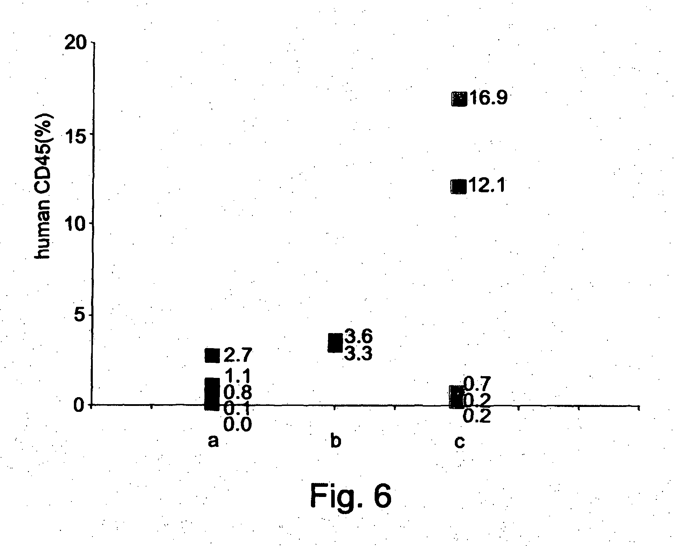

FIG. 6 is a graph depicting percentage of human CD45+ cells detected in bone marrow (BM) of NOD-SCID mice, treated with chemotherapy (25 mg/kg busulfan intraperitoneal injections for two consecutive weeks) 3.5 weeks following transplantation. CD34+ cells (100,000) purified from mononuclear cord blood derived cells, were transplanted alone (5 mice, a) or co-transplanted with 0.5 X 10⁶ placenta derived adherent cells cultured in 2D conditions (2D-ASC; 2 mice, b), or placenta derived adherent cells cultured in 3D conditions (3D-ASC), in the pluriX™ bioreactor (5 mice, c). BM was then collected from mice femurs and tibias. Human cells in the BM were detected by flow cytometry. The percentage of CD45 expressing human cells was determined by incubating cells with anti-human CD45-FITC. Note the higher percentage of human cells (hCD45+) in the bone marrow of mice co-transplanted with 2D-ASC (b) as well as with 3D-ASC (c) in comparison to the percentage of human cells in the mice treated with HSCs alone (a). The higher engraftment seen in mice treated with 3D-ASC cultured cells in comparison to mice treated with 2D-ASC cultured cells indicates a higher therapeutic advantage unique to 3D cultured ASCs.

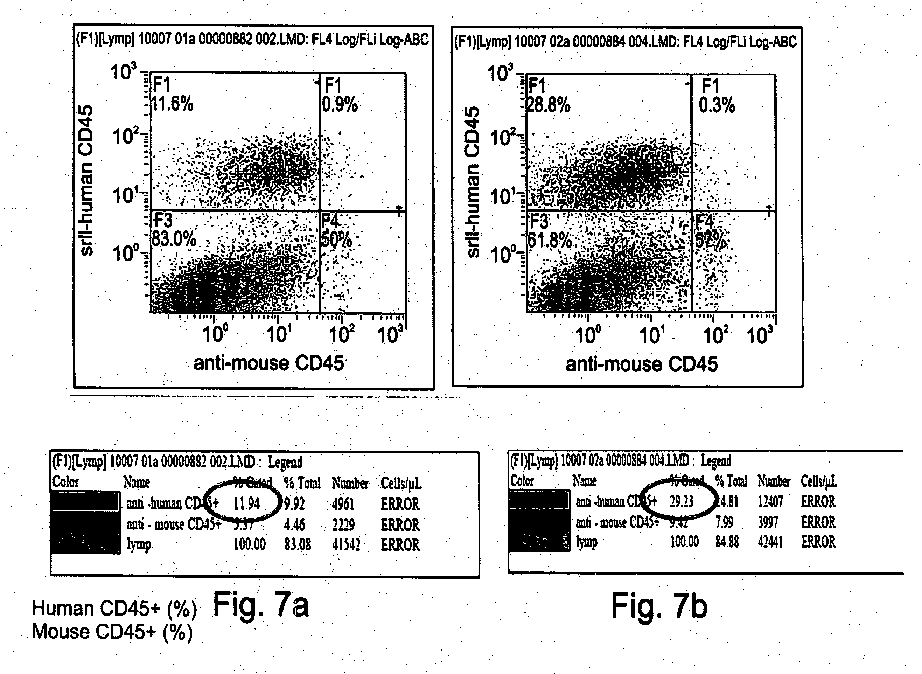

FIGs. 7a-b are FACS analyses of human graft CD45+ cells in mice transplanted with CD34+ cells only (Figure 7a) in comparison to CD34+ cells together with adipose tissue derived ASCs. (Figure 7b). Note the significantly higher percentage of human hematopoietic population (hCD45+) (7a - 29 %) in a mouse co-transplanted with adipose tissue derived ASC in comparison to a mouse treated with human CD34+ alone (7b -12 %).

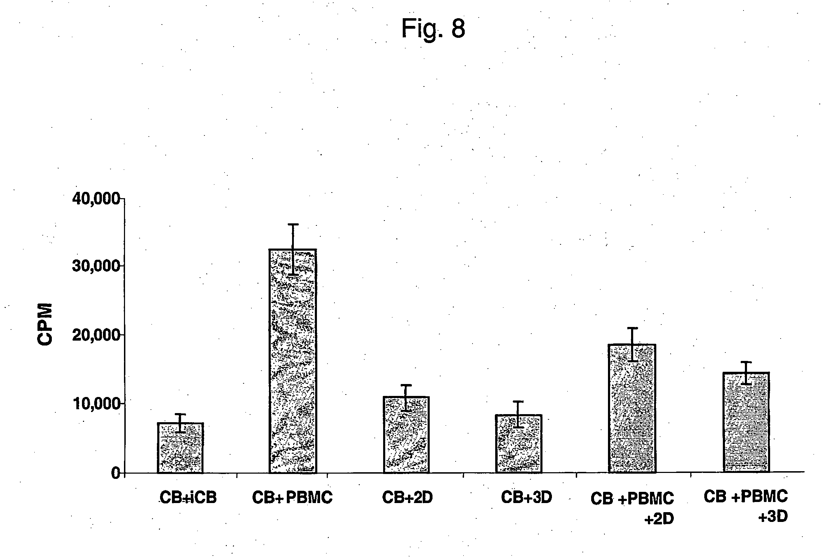

FIG. 8 is a bar graph depicting a mixed lymphocyte reaction conducted between human cord blood mononuclear cells (CB), and equal amounts of irradiated (3000 Rad) cord blood cells (iCB), human peripheral blood derived monocytes (PBMC), 2D cultured (2D) or 3D cultured (3D) placental ASCs, or a combination of PBMC and 2D and 3D cultured placental ASCs (PBMC+2D and PBMC+3D). Size of CB cell population is represented by the ³H-thymidine uptake (measured in CPM) which was measured during the last 18 hours of culturing. Elevation in stimulated CB cell proliferation indicates an immune response of a higher level. Note the lower level of immune response exhibited by cells incubated with adherent cells, and, in particular, the reduction of CB immune response to PBMCs when co-incubated with adherent cells. Three replicates were made of each reaction.

### DESCRIPTION OF THE PREFERRED EMBODIMENTS

The present invention is of novel methods of cell expansion and uses of cells and conditioned medium produced thereby, for stem cell related therapy, stem cell engraftment and HSC support.

The principles and operation of the present invention may be better understood with reference to the drawings and accompanying descriptions.

Before explaining at least one embodiment of the invention in detail, it is to be understood that the invention is not limited in its application to the details set forth in the following description or exemplified by the Examples. The invention is capable of other embodiments or of being practiced or carried out in various ways. Also, it is to be understood that the phraseology and terminology employed herein is for the purpose of description and should not be regarded as limiting.

In the developing medical world, there is a growing need for stem cells, and more specifically for stromal stem cells (also termed "mesenchymal stem cells"), for clinical and research purposes. MSCs are used for support of HSC transplantation and engraftment and also for curing a growing number of conditions e.g., heart diseases, BM deficiencies, neuronal related diseases, and conditions which require organ or tissue transplantation.

Obstacles in using stem cells lie in the technical difficulty of isolating large quantities of normally occurring populations of stem or progenitor cells, due to limited quantity of these cells in most tissues, the discomfort and risk involved in the procedures for obtaining stem cells, and the accompanying loss of memory B cells and hematopoietic stem cells with present harvesting procedures. Obtaining cells from the human embryo add a religious and ethical aspect to the already existing technical difficulties.

Alternative sources for bone marrow-derived stem cells include adipose tissues and placenta. However, currently there are no methods for efficient expansion of stem cells from such tissues.

While reducing the present invention to practice, the present inventors have uncovered that adherent cells from placenta or adipose tissue can be efficiently propagated in 3D culturing conditions. Surprisingly, the present inventors uncovered that such cells comprise functional properties which are similar to those of MSCs and therefore these cells and the conditioned medium produced there from, can be used for therapeutic purposes such as transplantation, tissue regeneration and *in vivo* HSC support.

As is illustrated herein below and in the Examples section which follows, the present inventors were able to expand adipose and placenta-derived adherent cells which comprise stromal stem cells properties in 3D settings. Cells expanded accordingly were found viable, following cryo-preservation, as evidenced by adherence and re-population assays (see Example 1). Flow cytometry analysis of placenta-derived adherent cells uncovered a distinct marker expression pattern and (see Figures 3a-b). Most importantly, adipose and placenta derived adherent cells propagated on 2D or 3D settings were able to support HSC engraftment (see Example 2), substantiating the use of the cells of the present invention, as stromal stem cells, in the clinic.

Thus, according to one aspect of the present invention, there is provided a method of cell expansion.

The method comprising culturing adherent cells from placenta or adipose tissue under three-dimensional (3D) culturing conditions which support cell expansion.

As used herein the terms "expanding" and "expansion" refer to substantially differentiationless maintenance of the cells and ultimately cell growth, i.e., increase of a cell population (e.g., at least 2 fold) without differentiation accompanying such increase.

As used herein the terms "maintaining" and "maintenance" refer to substantially differentiationless cell renewal, i.e., substantially stationary cell population without differentiation accompanying such stationarity.

As used herein the phrase "adherent cells" refers to a homogeneous or heterogeneous population of cells which are anchorage dependent, i.e., require attachment to a surface in order to grow *in vitro.*

As used herein the phrase "adipose tissue" refers to a connective tissue which comprises fat cells (adipocytes).

As used herein the term "placenta tissue" refers to any portion of the mammalian female organ which lines the uterine wall and during pregnancy envelopes the fetus, to which it is attached by the umbilical cord. Following birth, the placenta is expelled (and is referred to as a post partum placenta).

As used herein the phrase "three dimensional culturing conditions" refers to disposing the cells to conditions which are compatible with cell growth while allowing the cells to grow in more than one layer. It is well appreciated that the *in situ* environment of a cell in a living organism (or a tissue) as a three dimensional architecture. Cells are surrounded by other cells. They are held in a complex network of extra cellular matrix nanoscale fibers that allows the establishment of various local microenvironments. Their extra cellular ligands mediate not only the attachment to the basal membrane but also access to a variety of vascular and lymphatic vessels. Oxygen, hormones and nutrients are ferried to cells and waste products are carried away. The three dimensional culturing conditions of the present invention are designed to mimic such as environment as is further exemplified below.

Thus, adherent cells of this aspect of the present invention are retrieved from an adipose or placental tissue.

Placental cells may be obtained from a full-term or pre-term placenta. Placenta are preferably collected once it has been ex blooded. The placenta is preferably perfused for a period of time sufficient to remove residual cells. The term "perfuse" or "perfusion" used herein refers to the act of pouring or passaging a fluid over or through an organ or tissue. The placental tissue may be from any mammal; most preferably the plancental tissue is human. A convenient source of plancental tissue is from a post partum placenta (e.g., 1-6 hours), however, the source of plancental tissue or cells or the method of isolation of placental tissue is not critical to the invention.

Placenta derived adherent cells may be obtained from both fetal (i.e., amnion or inner parts of the placenta, see Example 1) and maternal (i.e., decidua basalis, and decidua parietalis) parts of the placenta. Tissue specimens are washed in a physiological buffer [e.g., phosphate-buffered saline (PBS) or Hank's buffer). Single-cell suspensions are made by treating the tissue with a digestive enzyme (see below) or/and mincing and flushing the tissue parts through a nylon filter or by gentle pipetting (Falcon, Becton, Dickinson, San Jose, CA) with washing medium.

Adipose tissue derived adherent cells may be isolated by a variety of methods known to those skilled in the art. For example, such methods are described in U.S. Pat. No. 6,153,432. The adipose tissue may be derived from omental/visceral, mammary, gonadal, or other adipose tissue sites. A preferred source of adipose tissue is omental adipose. In humans, the adipose is typically isolated by liposuction.

Isolated adherent cells from adipose tissue may be derived by treating the tissue with a digestive enzyme such as collagenase, trypsin and/or dispase; and/or effective concentrations of hyaluronidase or DNAse; and ethylenediaminetetra-acetic acid (EDTA); at temperatures between 25 - 50 °C, for periods of between 10 minutes to 3 hours. The cells may then be passed through a nylon or cheesecloth mesh filter of between 20 microns to 800 microns. The cells are then subjected to differential centrifugation directly in media or over a Ficoll or Percoll or other particulate gradient. Cells are centrifuged at speeds of between 100 to 3000 x g for periods of between 1 minutes to 1 hour at temperatures of between 4- 50 °C (see U.S. Pat. No. 7,078,230).

In addition to placenta or adipose tissue derived adherent cells, the present description also envisages the use of adherent cells from other cell sources which are characterized by stromal stem cell phenotype (as will be further described herein below). Tissue sources from which adherent cells can be retrieved include, but are not limited to, cord blood, hair follicles [e.g. as described in Us Pat. App. 20060172304], testicles [e.g., as described in Guan K., et al., Nature. 2006 Apr 27;440(7088):1199-203], human olfactory mucosa [e.g., as described in Marshall, CT., et al., Histol Histopathol. 2006 Jun;21(6):633-43], and amniotic fluid [Pieternella et al. (2004) Stem Cells 22:1338-1345], all of which are known to include mesenchymal stem cells. Adherent cells from these tissue sources can be isolated by culturing the cells on an adherent surface, thus isolating adherent cells from other cells in the initial population.

Regardless of the origin (e.g., placenta or adipose tissue), cell retrieval is preferably effected under sterile conditions. Once isolated cells are obtained, they are allowed to adhere to an adherent material (e.g., configured as a surface) to thereby isolate adherent cells. This may be effected prior to (see Example 1) or concomitant with culturing in 3D culturing conditions.

As used herein "an adherent material" refers to a synthetic, naturaly occurring or a combination of same of a non-cytotoxic (i.e., biologically compatible) material having a chemical structure (e.g., charged surface exposed groups) which may retain the cells on a surface.

Examples of adherent materials which may be used in accordance with this aspect of the present invention include, but are not limited to, a polyester, a polyalkylene, a polyfluorochloroethylene, a polyvinyl chloride, a polystyrene, a polysulfone, a cellulose acetate, a glass fiber, a ceramic particle, a matrigel, an extra cellular matrix component (e.g., fibronectin, chondronectin, laminin), a collagen, a poly L lactic acid and an inert metal fiber.

Further steps of purification or enrichment for stromal stem cells may be effected using methods which are well known in the art (such as by FACS using stromal stem cell marker expression, as further described herein below).

Non-limiting examples of base media useful in culturing according to the present invention include Minimum Essential Medium Eagle, ADC-1, LPM (Bovine Serum Albumin-free), F10(HAM), F12 (HAM), DCCM1, DCCM2, RPMI 1640, BGJ Medium (with and without Fitton-Jackson Modification), Basal Medium Eagle (BME-with the addition of Earle's salt base), Dulbecco's Modified Eagle Medium (DMEM-without serum), Yamane, IMEM-20, Glasgow Modification Eagle Medium (GMEM), Leibovitz L-15 Medium, McCoy's 5A Medium, Medium M199 (M199E-with Earle's sale base), Medium M199 (M199H-with Hank's salt base), Minimum Essential Medium Eagle (MEM-E-with Earle's salt base), Minimum Essential Medium Eagle (MEM-H-with Hank's salt base) and Minimum Essential Medium Eagle (MEM-NAA with non essential amino acids), among numerous others, including medium 199, CMRL 1415, CMRL 1969, CMRL 1066, NCTC 135, MB 75261, MAB 8713, DM 145, Williams' G, Neuman & Tytell, Higuchi, MCDB 301, MCDB 202, MCDB 501, MCDB 401, MCDB 411, MDBC 153. A preferred medium for use in the present invention is DMEM. These and other useful media are available from GIBCO, Grand Island, N.Y., USA and Biological Industries, Bet HaEmek, Israel, among others. A number of these media are summarized in Methods in Enzymology, Volume LVIII, "Cell Culture", pp. 62 72, edited by William B. Jakoby and Ira H. Pastan, published by Academic Press, Inc.

The medium may be supplemented such as with serum such as fetal serum of bovine or other species, and optionally or alternatively, growth factors, cytokines, and hormones (e.g., growth hormone, erythropoeitin, thrombopoietin, interleukin 3, interleukin 6, interleukin 7, macrophage colony stimulating factor, c-kit ligand/stem cell factor, osteoprotegerin ligand, insulin, insulin like growth factors, epidermal growth factor, fibroblast growth factor, nerve growth factor, cilary neurotrophic factor, platelet derived growth factor, and bone morphogenetic protein at concentrations of between pigogram/ml to milligram/ml levels.

It is further recognized that additional components may be added to the culture medium. Such components may be antibiotics, antimycotics, albumin, amino acids, and other components known to the art for the culture of cells. Additionally, components may be added to enhance the differentiation process when needed (see further below).

Once adherent cells are at hand they may be passaged to three dimensional settings (see Example 1 of the Examples section which follows). It will be appreciated though, that the cells may be transferred to a 3D-configured matrix immediately after isolation (as mentioned hereinabove).

Thus, the adherent material of this aspect of the present invention is configured for 3D culturing thereby providing a growth matrix that substantially increases the available attachment surface for the adherence of the stromal cells so as to mimic the infrastructure of the tissue (e.g., placenta).

For example, for a growth matrix of 0.5 mm in height, the increase is by a factor of at least from 5 to 30 times, calculated by projection onto a base of the growth matrix. Such an increase by a factor of about 5 to 30 times, is per unit layer, and if a plurality of such layers, either stacked or separated by spacers or the like, is used, the factor of 5 to 30 times applies per each such structure. When the matrix is used in sheet form, preferably non-woven fiber sheets, or sheets of open-pore foamed polymers, the preferred thickness of the sheet is about 50 to 1000 µm or more, there being provided adequate porosity for cell entrance, entrance of nutrients and for removal of waste products from the sheet. According to a preferred embodiment the pores have an effective diameter of 10 µm to 100 µm. Such sheets can be prepared from fibers of various thicknesses, the preferred fiber thickness or fiber diameter range being from about 0.5 µm to 20 µm, still more preferred fibers are in the range of 10 µm to 15 µm in diameter.

The structures of the invention may be supported by, or even better bonded to, a porous support sheet or screen providing for dimensional stability and physical strength.

Such matrix sheets may also be cut, punched, or shredded to provide particles with projected area of the order of about 0.2 mm² to about 10 mm², with the same order of thickness (about 50 to 1000 µm).

Further details relating to the fabrication, use and/or advantages of the growth matrix which was used to reduce the present invention to practice are described in U.S. Pat Nos. 5,168,085, and in particular, 5,266,476.

The adherent surface may have a shape selected from the group consisting of squares, rings, discs, and cruciforms.

For high scale production, culturing is preferably effected in a 3D bioreactor.

Examples of such bioreactors include, but are not limited to, a plug flow bioreactor, a continuous stirred tank bioreactor and a stationary-bed bioreactor.

As shown Example 1 of the Examples section, a three dimensional (3D) plug flow bioreactor (as described in US Pat. No. 6911201) is capable of supporting the growth and prolonged maintenance of stromal cells. In this bioreactor, stromal cells are seeded on porrosive carriers made of a non woven fabric matrix of polyester, packed in a glass column, thereby enabling the propagation of large cell numbers in a relatively small volume.

The matrix used in the plug flow bioreactor can be of sheet form, non-woven fiber sheets, or sheets of open-pore foamed polymers, the preferred thickness of the sheet is about 50 to 1000 µm or more, there being provided adequate porosity for cell entrance, entrance of nutrients and for removal of waste products from the sheet.

Other 3D bioreactors that can be used with the present invention include, but are not limited to, a continuous stirred tank bioreactor, where a culture medium is continuously fed into the bioreactor and a product is continuously drawn out, to maintain a time-constant steady state within the reactor]. A stirred tank bioreactor with a fibrous bed basket is available for example at New Brunswick Scientific Co., Edison, NJ), A stationary-bed bioreactor, an air-lift bioreactor, where air is typically fed into the bottom of a central draught tube flowing up while forming bubbles, and disengaging exhaust gas at the top of the column], a cell seeding perfusion bioreactor with Polyactive foams [as described in Wendt, D. et al., Biotechnol Bioeng 84: 205-214, (2003)] tubular poly-L-lactic acid (PLLA) porous scaffolds in a Radial-flow perfusion bioreactor [as described in Kitagawa et al., Biotechnology and Bioengineering 93(5): 947-954 (2006). Other bioreactors which can be used in accordance with the present invention are described in U.S. Pat. Nos. 6,277,151, 6,197,575,6,139,578, 6,132,463, 5,902,741 and 5,629,186.

Cell seeding is preferably effected 100,000-1,500,000 cells / mm at seeding.

Cells are preferably harvested once reaching at least about 40 % confluence, 60 % confluence or 80 % confluence while preferably avoiding uncontrolled differentiation and senescence.

Culturing is effected for at least about 2 days, 3 days, 5 days, 10 days, 20 days, a month or even more. It will be appreciated that culturing in a bioreactor may prolong this period. Passaging may also be effected to increase cell number.

Adherent cells of the present invention preferably comprise at least one "stromal stem cell phenotype".

As used herein "a stromal stem cell phenotype" refers to a structural or functional phenotype typical of a bone-marrow derived stromal (i.e., mesenchymal) stem cell

As used herein the phrase "stem cell" refers to a cell which is not terminally differentiated.

Thus for example, the cells may have a spindle shape. Alternatively or additionally the cells may express a marker or a collection of markers (e.g. surface marker) typical to stromal stem cells. Examples of stromal stem cell surface markers (positive and negative) include but are not limited to CD105+, CD29+, CD44+, CD73+, CD90+, CD34-, CD45-, CD80-, CD19-, CD5-, CD20-, CD11B-, CD14-, CD19-, CD79-, HLA-DR-, and FMC7-. Other stromal stem cell markers include but are not limited to tyrosine hydroxylase, nestin and H-NF.

Examples of functional phenotypes typical of stromal stem cells include, but are not limited to, T cell suppression activity (don't stimulate T cells and conversely suppress same), hematopoietic stem cell support activity, as well as adipogenic, hepatogenic, osteogenic and neurogenic differentiation.

Any of these structural or functional features can be used to qualify the cells of the present invention (see Examples 1-2 of the Examples section which follows).

Populations of cells generated according to the present teachings are characterized by a unique protein expression profile as is shown in Example 1 of the Examples section. Thus for example, adherent cells of placenta or adipose tissue generated according to the present teachings, are capable of expressing and/or secreting high levels of selected factors. For example, such cells express or secrete SCF, Flt-3, H2AF or ALDH X at least 2, 3, 4, 5, 6, 7, 8, 9, 10, 11 or preferably 12 fold higher than that expressed or secreted by adherent cells of placenta or adipose tissue grown in a 2D culture. Additionally or alternatively, population of cells of the present invention secrete or express IL-6, EEEF2, RCN2 or CNN1 at a level least 2, 3 or 5 fold higher than that expressed or secreted by adherent cells of placenta or adipose tissue grown in a 2D culture. Additionally or alternatively, population of cells of the present invention are characterized by lower level of expression of various other proteins as compared to 2D cultured cells. Thus for example, secrete or express less than 0.6, 0.5, 0.25 or 0.125 of the expression level of Hnrph1, CD44 antigen isoform 2 precursor, Papss2 or rpL7a expressed or secreted by adherent cells of placenta or adipose tissue grown in a 2D culture.

While further reducing the present invention to practice the present inventors have realized that adherent stromal cells, and particularly 3D-ASCs, showed immunosuppressive activity. As is shown in Example 3 of the Examples section which follows, Adherent stromal cells, and particularly 3D-ASCs, were found to suppress the immune reaction of human cord blood mononuclear cells in an MLR assay. Thus, the cells of the present invention may comprise biological activities which may be preferentially used in the clinic (e.g., T cell suppression activity, hematopoietic stem cell support activity).

While further reducing the present invention to practice the present inventors have realized that conditioned medium of the cells of the present invention may comprise biological activities which may be preferentially used in the clinic (e.g., T cell suppression activity, hematopoietic stem cell support activity).

Thus, the present invention further envisages collection of conditioned medium and its use as is or following further steps of concentration, enrichment or fractionation using methods which are well known in the art. Preferably a conditioned medium of the present is obtained from a high viability mid-log culture of cells.

As mentioned hereinabove, cells and conditioned media of the present invention are characterized by a stromal stem cell phenotype and as such can be used in any research and clinical application which may benefit from the use of such cells.

Engraftment and initiation of hematopoiesis by transplanted HSCs depend on complex processes which include homing, following a gradient of chemokines across the endothelial cell barrier, to the bone marrow and lodging in the appropriate niches, while establishing physical contacts between transplanted cells, the ECM and the mesenchymal cells of the niches. All these processes involve a complex array of molecules, such as cytokines, hormones, steroids, extra cellular matrix proteins, growth factors, cell-to-cell interaction and adhesion proteins, and matrix proteins.

It is known that only 1-5 % of transfused HSCs are detected in the recipient BM 2-3 days post transplantation [Kerre et al., J Immunol. 167:3692-8. (2001); Jetmore et al., Blood. 99:1585-93 (2002)].

MSCs contribution to hematopoietic engraftment is in part by the inhibition of donor derived T cell production, which cause graft vs. host disease [GvHD, Charbord P., and Moore, K., Ann. N.Y. Acad. Sci. 1044: 159-167 (2005); Maitra B, et al., Bone Marrow Transplant. 33(6):597-604. (2004); US patent nos. 6,010,696; 6555374]; and part by providing a hematopoietic stem cell (HSC) support (i.e., sustaining and aiding the proliferation, maturation and/or homing of hematopoietic stem cells).

As shown in Example 2 of the Examples section which follows, placenta and adipose tissue-derived adherent cells were surprisingly found to be supportive of HSC engraftment even after chemotherapy.

Given these results it is conceivable that cells or media of the present invention may be used in any clinical application for which stromal stem cell transplantation is used.

Thus, according to another aspect of the present invention there is provided a method of treating a medical condition (e.g., pathology, disease, syndrome) which may benefit from stromal stem cell transplantation in a subject in need thereof.

As used herein the term "treating" refers to inhibiting or arresting the development of a pathology and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology. Preferably, the term "treating" refers to alleviating or diminishing a symptom associated with a cancerous disease. Preferably, treating cures, e.g., substantially eliminates, the symptoms associated with the medical condition.

As used herein "a medical condition which may benefit from stromal stem cell transplantation" refers to any medical condition which may be alleviated by administration of cells/media of the present invention.

The term or phrase "transplantation", "cell replacement" or "grafting" are used interchangeably herein and refer to the introduction of the cells of the present invention to target tissue.

As used herein the term "subject" refers to any subject (e.g., mammal), preferably a human subject.

The method of this aspect of the present invention comprises administering to the subject a therapeutically effective amount of the cells or media of the present invention (described hereinabove), thereby treating the medical condition which may benefit from stromal stem cell transplantation in the subject

Cells which may be administered in accordance with this aspect of the present invention include the above-described adherent cells which may be cultured in three-dimensional settings as well as mesenchymal and-non mesenchymal partially or terminally differentiated derivatives of same.

Methods of deriving lineage specific cells from the stromal stem cells of the present invention are well known in the art. See for example, U.S. Pat. Nos. 5,486,359, 5,942,225, 5,736,396, 5,908,784 and 5,902,741.

The cells may be naïve or genetically modified such as to derive a lineage of interest (see U.S. Pat. Appl. No. 20030219423).

The cells and media may be of autologous or non-autologous source (i.e., allogenic or xenogenic) of fresh or frozen (e.g., cryo-preserved) preparations.

Depending on the medical condition, the subject may be administered with additional chemical drugs (e.g., immunomodulatory, chemotherapy etc.) or cells.

Thus, for example, for improving stem cell engraftment (e.g., increasing the number of viable HSC in the recipient BM and optimally improve normal white blood cell count) the cells/media of the present invention may be administered prior to, concomitantly with or following HSC transplantation.

Preferably the HSCs and stromal cells share common HLA antigens. Preferably, the HSCs and stromal cells are from a single individual. Alternatively, the HSCs and stromal cells are from different individuals.

The term or phrase "transplantation", "cell replacement" or "grafting" are used interchangeably herein and refer to the introduction of the cells of the present invention to target tissue. The cells can be derived from the recipient or from an allogeneic or xenogeneic donor.

Since non-autologous cells are likely to induce an immune reaction when administered to the body several approaches have been developed to reduce the likelihood of rejection of non-autologous cells. These include either suppressing the recipient immune system or encapsulating the non-autologous cells in immunoisolating, semipermeable membranes before transplantation.

Encapsulation techniques are generally classified as microencapsulation, involving small spherical vehicles and macroencapsulation, involving larger flat-sheet and hollow-fiber membranes (Uludag, H. et al. Technology of mammalian cell encapsulation. Adv Drug Deliv Rev. 2000; 42: 29-64).

Methods of preparing microcapsules are known in the arts and include for example those disclosed by Lu MZ, et al., Cell encapsulation with alginate and alpha-phenoxycinnamylidene-acetylated poly(allylamine). Biotechnol Bioeng. 2000, 70: 479-83, Chang TM and Prakash S. Procedures for microencapsulation of enzymes, cells and genetically engineered microorganisms. Mol Biotechnol. 2001, 17: 249-60, and Lu MZ, et al., A novel cell encapsulation method using photosensitive poly(allylamine alpha-cyanocinnamylideneacetate). J Microencapsul. 2000, 17: 245-51.

For example, microcapsules are prepared by complexing modified collagen with a ter-polymer shell of 2-hydroxyethyl methylacrylate (HEMA), methacrylic acid (MAA) and methyl methacrylate (MMA), resulting in a capsule thickness of 2-5 µm. Such microcapsules can be further encapsulated with additional 2-5 µm ter-polymer shells in order to impart a negatively charged smooth surface and to minimize plasma protein absorption (Chia, S.M. et al. Multi-layered microcapsules for cell encapsulation Biomaterials. 2002 23: 849-56).

Other microcapsules are based on alginate, a marine polysaccharide (Sambanis, A. Encapsulated islets in diabetes treatment. Diabetes Technol. Ther. 2003, 5: 665-8) or its derivatives. For example, microcapsules can be prepared by the polyelectrolyte complexation between the polyanions sodium alginate and sodium cellulose sulphate with the polycation poly(methylene-co-guanidine) hydrochloride in the presence of calcium chloride.

It will be appreciated that cell encapsulation is improved when smaller capsules are used. Thus, the quality control, mechanical stability, diffusion properties, and *in vitro* activities of encapsulated cells improved when the capsule size was reduced from 1 mm to 400 µm (Canaple L. et al., Improving cell encapsulation through size control. J Biomater Sci Polym Ed. 2002;13:783-96). Moreover, nanoporous biocapsules with well-controlled pore size as small as 7 nm, tailored surface chemistries and precise microarchitectures were found to successfully immunoisolate microenvironments for cells (Williams D. Small is beautiful: microparticle and nanoparticle technology in medical devices. Med Device Technol. 1999, 10: 6-9; Desai, T.A. Microfabrication technology for pancreatic cell encapsulation. Expert Opin Biol Ther. 2002, 2: 633-46).

Examples of immunosuppressive agents include, but are not limited to, methotrexate, cyclophosphamide, cyclosporine, cyclosporin A, chloroquine, hydroxychloroquine, sulfasalazine (sulphasalazopyrine), gold salts, D-penicillamine, leflunomide, azathioprine, anakinra, infliximab (REMICADE), etanercept, TNF.alpha. blockers, a biological agent that targets an inflammatory cytokine, and Non-Steroidal Anti-Inflammatory Drug (NSAIDs). Examples of NSAIDs include, but are not limited to acetyl salicylic acid, choline magnesium salicylate, diflunisal, magnesium salicylate, salsalate, sodium salicylate, diclofenac, etodolac, fenoprofen, flurbiprofen, indomethacin, ketoprofen, ketorolac, meclofenamate, naproxen, nabumetone, phenylbutazone, piroxicam, sulindac, tolmetin, acetaminophen, ibuprofen, Cox-2 inhibitors and tramadol.

In any of the methods described herein, the cells or media can be administered either *per se* or, preferably as a part of a pharmaceutical composition that further comprises a pharmaceutically acceptable carrier.

As used herein a "pharmaceutical composition" refers to a preparation of one or more of the chemical conjugates described herein, with other chemical components such as pharmaceutically suitable carriers and excipients. The purpose of a pharmaceutical composition is to facilitate administration of a compound to a subject.

Hereinafter, the term "pharmaceutically acceptable carrier" refers to a carrier or a diluent that does not cause significant irritation to a subject and does not abrogate the biological activity and properties of the administered compound. Examples, without limitations, of carriers are propylene glycol, saline, emulsions and mixtures of organic solvents with water.

Herein the term "excipient" refers to an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

According to a preferred embodiment of the present invention, the pharmaceutical carrier is an aqueous solution of saline.

Techniques for formulation and administration of drugs may be found in "Remington's Pharmaceutical Sciences," Mack Publishing Co., Easton, PA, latest edition.

One may administer the pharmaceutical composition in a systemic manner (as detailed hereinabove). Alternatively, one may administer the pharmaceutical composition locally, for example, via injection of the pharmaceutical composition directly into a tissue region of a patient.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For any preparation used in the methods of the invention, the therapeutically effective amount or dose can be estimated initially from *in vitro* and cell culture assays. Preferably, a dose is formulated in an animal model to achieve a desired concentration or titer. Such information can be used to more accurately determine useful doses in humans.

Toxicity and therapeutic efficacy of the active ingredients described herein can be determined by standard pharmaceutical procedures *in vitro,* in cell cultures or experimental animals.

The data obtained from these *in vitro* and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition, (see e.g., Fingl, et al., 1975, in "The Pharmacological Basis of Therapeutics", Ch. 1 p.1). For example, Parkinson's patient can be monitored symptomatically for improved motor functions indicating positive response to treatment.

For injection, the active ingredients of the pharmaceutical composition may be formulated in aqueous solutions, preferably in physiologically compatible buffers such as Hank's solution, Ringer's solution, or physiological salt buffer.

Dosage amount and interval may be adjusted individually to levels of the active ingredient which are sufficient to effectively regulate the neurotransmitter synthesis by the implanted cells. Dosages necessary to achieve the desired effect will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or diminution of the disease state is achieved.

The amount of a composition to be administered will, of course, be dependent on the individual being treated, the severity of the affliction, the manner of administration, the judgment of the prescribing physician, etc. The dosage and timing of administration will be responsive to a careful and continuous monitoring of the individual changing condition. For example, a treated Parkinson's patient will be administered with an amount of cells which is sufficient to alleviate the symptoms of the disease, based on the monitoring indications.

Following transplantation, the cells of the present invention preferably survive in the diseased area for a period of time (e.g. at least 6 months), such that a therapeutic effect is observed.

Compositions including the preparation of the present invention formulated in a compatible pharmaceutical carrier may also be prepared, placed in an appropriate container, and labeled for treatment of an indicated condition.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert.

### EXAMPLES

Reference is now made to the following examples, which together with the above descriptions illustrate the invention in a non-limiting fashion.

Generally, the nomenclature used herein and the laboratory procedures utilized in the present invention include molecular, biochemical, microbiological andrecombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); forth herein. Other general references are provided throughout this document. The procedures therein are believed to be well known in the art and are provided for the convenience of the reader. All the information contained therein is incorporated herein by reference.

### EXAMPLE 1

### Production and culturing of adherent stromal cells (ASC) from bone marrow, placenta and adipose tissues

Adherent cells were cultured in a bioreactor system containing 3D carriers to produce 3D-ASC cells, characterized by a specific cell marker expression profile. Growth efficiency was tested through cell count. The differentiation capacity of these cells was tested by culturing in a differentiation medium.

### MATERIALS AND EXPERIMENTAL PROCEDURES

***Bone marrow stromal cells -*** Bone marrow (BM) stromal cells were obtained from aspirated sterna marrow of hematologically healthy donors undergoing open-heart surgery or BM biopsy. Marrow aspirates were diluted 3-fold in Hank's Balanced Salts Solution (HBSS; GIBCO BRL/Invitrogen, Gaithersburg MD) and subjected to Ficoll-Hypaque (Robbins Scientific Corp. Sunnyvale, CA) density gradient centrifugation. Thereafter, marrow mononuclear cells (<1.077 gm/cm3) were collected, washed 3 times in HBSS and resuspended in growth media [DMEM (Biological Industries, Beit Ha'emek, Israel) supplemented with 10 % FCS (GIBCO BRL), 10⁻⁴ M mercaptoethanol (Merck, White House Station, NJ), Pen-Strep-Nystatin mixture (100 U/ml:100 ug/ml:1.25 un/ml; Beit Ha'Emek), 2 mM L-glutamine (Beit Ha'Emek)]. Cells from individual donors were incubated separately in tissue culture flasks (Corning, Acton, MA) at 37 °C (5 % CO₂) with weekly change of culture media. Cells were split every 3-4 days using 0.25 % trypsin-EDTA (Beit Ha'Emek). Following 2-40 passages, when reaching 60-80 % confluence, cells were collected for analysis or for culturing in bioreactors.

***Placenta derived stromal cells -*** Inner parts of a full-term delivery placenta (Bnei Zion medical center, Haifa, Israel) were cut under sterile conditions, washed 3 times with Hank's Buffer and incubated for 3 h at 37°C with 0.1 % Collagenase (1mg / ml tissue; Sigma- Aldrich, St. Lewis, MO). Using gentle pipeting, suspended cells were then washed with DMEM supplemented with 10 % FCS, Pen-Strep-Nystatin mixture (100 U/ml:100 ug/ml:1.25 un/ml) and 2 mM L-glutamine, seeded in 75 cm² flasks and incubated at 37 °C in a tissue culture incubator under humidified condition with 5 % CO₂. Thereafter, cells were allowed to adhere to a plastic surface for 72 hours after which the media was changed every 3 - 4 days. When reaching 60-80 % confluence (usually 10-12 days), cells were detached from the growth flask using 0.25 % trypsin-EDTA and seeded into new flasks. Cultured cells were thereafter collected for analysis or for culturing in bioreactors.

***Adipose derived stromal cells* -** Stromal cells were obtained from human adipose tissue of liposuction procedures (Rambam Haifa, Israel). Adipose tissue was washed extensively with equal volumes of PBS and digested at 37 °C for 30 min with collagenase (20 mg/ml). Cells were then washed with DMEM containing 10 % FCS, Pen-Strep-Nystatin mixture (100 U/ml:100 ug/ml:1.25 un/ml) and L- Glutamin and centrifuged at 1200 rpm for 10 min RT, resuspended with lysing solution (1:10; Biological Industries, Beit Ha'emek, Israel, in order to discard red-blood cells) centrifuged and resuspended with DMEM containing 10 % FCS, Pen-Strep-Nystatin mixture (100 U/ml:100 ug/ml:1.25 un/ml) and L- Glutamin. Washed cells were then seeded in a sterile tissue culture medium flask at 3-10 X 10⁷ cells/flask. At the next day cells were washed with PBS to remove residual RBC and dead cells. The cells were kept at 37 °C in a tissue culture incubator under humidified condition with 5 % CO₂. The medium was changed every 3 to 4 days. At 60-80 % confluence, the cells were detached from the growth flask using 0.25 % trypsin-EDTA and seeded into new flasks. Following 2-40 passages, when cells reached 60-80 % confluece, cells were collected for analysis or for culturing in bioreactors.

***PluriX™ Plug Flow bioreactor -*** The PluriX™ Plug Flow bioreactor (Pluristem, Haifa, Israel; as illustrated in Figure 1g, see also U.S. Pat. No. 6,911,201), was loaded with 1-100 ml packed 3D porrosive carriers (4 mm in diameter) made of a non woven fabric matrix of polyester. These carriers enable the propagation of large cell numbers in a relatively small volume. Glassware was designed and manufactured by Pluristem. The bioreactor was maintained in an incubator of 37 °C, with flow rate regulated and monitored by a valve (6a in Figure 1g), and peristaltic pump (9 in Figure 1g). The bioreactor contains a sampling and injection point (4 in Figure 1g), allowing the sequential seeding of cells. Culture medium was supplied at pH 6.7-7.4 from a reservoir (1 in Figure 1g). The reservoir was supplied by a filtered gas mixture (2,3 in Figure 1g), containing air/CO₂/O₂ at differing proportions, depending on cell density in the bioreactor. The O₂ proportion was suited to the level of dissolved O₂ at the bioreactor exit, determined by a monitor (6 in Figure 1g). The gas mixture was supplied to the reservoir via silicone tubes or diffuser (Degania Bet, Emek Hayarden, Israel). The culture medium was passed through a separating container (7 in Figure 1g) which enables collection of circulating, nonadherent cells. Circulation of the medium was obtained by a peristaltic pump (9 in Figure 1g). The bioreactor was further equipped with an additional sampling point (10 in Figure 1g) and containers for continuous medium exchange.

***Production of 3D-adherent stromal cells (3D-ASC)*** - Non-confluent primary human adherent 2D cell cultures, grown as described above, were trypsinized, washed, resuspended in DMEM supplemented with 10 % FBS, Pen-Strep-Nystatin mixture (100 U/ml:100 ug/ml:1.25 un/ml) and 2 mM L-glutamine, and seeded (10³-10⁵ cells/ml) via an injection point onto the 3D carriers in a sterile Plug Flow bioreactor (see Figure 1g). Prior to inoculation, bioreactor was filled with PBS-Ca-Mg (Biological Industries, Beit Ha'emek, Israel), autoclaved (120 °C, 30 min) and washed with Dulbecco's growth medium containing 10 % heat-inactivated fetal calf serum and a Pen-Strep-Nystatin mixture (100 U/ml:100 ug/ml:1.25 un/ml). Flow was kept at a rate of 0.1-5 ml/min. Seeding process involved cease of circulation for 2- 48 hrs, thereby allowing the cells to settle on the carriers. Bioreactor was kept under controlled temperature (37 °C) and pH conditions (pH = 6.7-7.4); using an incubator supplied with sterile air and CO₂ as needed. Growth medium was replaced 2-3 times a week. Circulation medium was replaced with fresh DMEM media, every 4 hr to 7 days. At a density of 1 X 10⁶-1 X 10⁷ cells/ml (following 12-40 days of growth), total medium volume was removed from the bioreactor and bioreactor and carriers were washed 3-5 times with PBS. 3D-ASC cells were then detached from the carriers with Trypsin-EDTA; (Biological Industries, Beit Ha'emek, Israel; 3-15 minutes with gentle agitation, 1-5 times), and were thereafter resuspended in DMEM and cryopreserved.

***3D-ASC quality biological assays -*** Cryopreserved 3D-ASC cells were thawed and counted. For cell viability evaluation, 2 X 10⁵ cells were seeded in a 150 cm² tissue culture flask and their adherence capability and repopulation was evaluated within 7 days following seeding. Thereafter, the 3D-ASC membrane marker phenotype was analyzed using fluorescence monoclonal antibodies flow-cytometer (Beckman Coulter, Fullerton, CA).

***Comparison between the cell membrane marker profile of 3D and 2D cultured adherent cells using flow cytometery assays*** 100,000 - 200,000 adherent cells from 2D cultures and 3D flow system cultures were suspended in 0.1 ml of culture medium in a 5 ml tube and incubated (4 °C, 30min, dark conditions) with saturating concentrations of each of the following MAbs: FITC-conjugated anti-human CD90 (Chemicon International Inc. Temecula, CA), PE conjugated anti human CD73 (Bactlab Diagnostic, Ceasarea, Israel), PE conjugated anti human CD105 (eBioscience, San Diego, CA), FITC conjugated anti human CD29 (eBioscience, San Diego, CA), Cy7-PE conjugated anti-human CD45 (eBiosience), PE-conjugated anti-human CD19 (IQProducts, Groningen, The Netherlands), PE conjugated anti human CD14 MAb (IQProducts), FITC conjugated anti human CD11b (IQProducts) and PE conjugated anti human CD34 (IQProducts) or with FITC conjugated anti human HLA-DR MAb (IQProducts). Following incubation the cells were washed twice in ice-cold PBS containing 1 % heat-inactivated FCS, resuspended in 500 µl formaldehyde 0.5 % and analyzed using the FC-500 flow-cytometer (Beckman Coulter, Fullerton, CA).

***Comparison between the protein profile of 3D and 2D cultured adherent cells using mass spectrometry analysis -*** 2D and 3D derived culturing procedures ASCs were produced from the placenta as described above. Briefly, the 2D cultures were produced by culturing 0.3-0.75 X 10⁶ cells in 175 cm² flasks for 4 days under humidified 5 % CO₂ atmosphere at 37 °C, until reaching 60 - 80 % confluence. The 3D cultures were produced by seeding 2-10 X 10⁶ cells/gram in a bioreactor containing 2000 carriers, and culturing for 18 days. Following harvesting, cells were washed (X 3) to remove all the serum, pelleted and frozen. Proteins were isolated from pellets [using Tri Reagent kit (Sigma, Saint Louis, USA) and digested with trypsin and labeled with iTRAQ reagent (Applied Biosciences, Foster City, CA)], according to the manufacturers protocol. Breifly, iTRAQ reagents are non-polymeric; isobaric tagging reagents. Peptides within each sample are labeled with one of four isobaric, isotope-coded tags via their N-terminal and/or lysine side chains. The four labeled samples are mixed and peptides are analyzed with mass spectrometery. Upon peptide fragmentation, each tag releases a distinct mass reporter ion; the ratio of the four reporters therefore gives relative abundances of the given peptide in a sample. (information at: http://docs.appliedbiosystems.com/pebiodocs/00113379.pdf).

Proteomics analysis of 2D culture versus 3D culture of placenta derived ASCs was performed in the Smoler proteomic center (department of Biology, Technion, Haifa, Israel) using LC-MS/MS on QTOF-Premier (Waters, San Francisco, CA), with identification and analysis done by Pep-Miner software [Beer, I., et al., Proteomics, 4, 950-60 (2004)] against the human part of the nr database. The proteins analyzed were: heterogeneous nuclear ribonucleoprotein H1 (Hnrph1 GeneBank Accession No. NP_005511), H2A histone family (H2AF, GeneBank Accession No. NP_034566.1), eukaryotic translation elongation factor 2 (EEEF2, GeneBank Accession No. NP_031933.1), reticulocalbin 3, EF-hand calcium binding domain (RCN2, GeneBank Accession No. NP_065701), CD44 antigen isoform 2 precursor (GeneBank Accession No. NP_001001389, calponin 1 basic smooth muscle (CNN1, GeneBank Accession No. NP_001290), 3 phosphoadenosine 5 phosphosulfate synthase 2 isoform a (Papss2, GeneBank Accession No. NP_004661), ribosomal protein L7a (rpL7a, GeneBank Accession No. NP_000963) and Aldehyde dehydrogenase X (ALDH X, GeneBank Accession No. P47738). Every experiment was done twice. Because of the nature of the analysis, every protein was analyzed according to the number of peptides of which appeared in a sample (2-20 appearances of a protein in each analysis)

***Comparison between secreted proteins in 3D and 2D cultured adherent cells using ELISA -*** 2D and 3D derived culturing procedures ASCs produced from the placenta, were produced as described above, with 3D cultures for the duration of 24 days. Conditioned media were thereafter collected and analyzed for Flt-3 ligand, IL-6, Trombopoietin (TPO) and stem cell factor (SCF), using ELISA (R&D Systems, Minneapolis, MN), in three independent experiments. Results were normalized for 1 X 10⁶ cells/ml.

***Osteoblast differentiating medium-*** Osteogenic differentiation was assessed by culturing of cells in an osteoblast differentiating medium consisting DMEM supplemented with 10 % FCS, 100 nM dexamethasone, 0.05 mM ascorbic acid 2-phosphate, 10 mM B-glycerophosphate, for a period of 3 weeks. Calcified matrix was indicated by Alizzarin Red S staining and Alkaline phosphatase was detected by Alkaline phosphatase assay kit (all reagents from Sigma- Aldrich, St. Lewis, MO).

### RESULTS

### The PluriX™ Bioreactor System creates a physiological -like microenvironment.

In order to render efficient culture conditions for adherent cells, a physiological -like environment (depicted in Figure 1a) was created artificially, using the PluriX Bioreactor (Pluristem, Haifa, Israel; carrier is illustrated in Figure 1g and shown before seeding in Figure 1b). As is shown in Figures 1c-f, bone marrow produced 3D-ASC cells were cultured successfully and expanded on the 3D matrix, 20 days (Figures 1b-c, magnified X 150 and 250 respectively) and 40 days (Figures 1c-d, magnified X 350 and 500 respectively) following seeding.

***Cells grown in the PluriX Bioreactor system were significantly expanded-***Different production lots of placenta derived 3D-ASC cells were grown in the PluriX bioreactor systems. The seeding density was 13,300 cells/carrier (to a total of 2 X 10⁶ cells). Fourteen days following seeding, cell density multiplied by 15 fold, reaching approximately 200,000 cells/carrier (Figure 2), or 30 X 10⁶ in a bioreactor of 150 carriers. In a different experiment, cells were seeded into the bioreactor at density of 1.5 X 10⁴ cells/ml and 30 days following seeding the carriers contained an over 50-fold higher cell number, i.e. approx. 0.5 X 10⁶ cells/carrier, or 0.5 X 10⁷ cells/ml. The cellular density on the carriers at various levels of the growth column was consistent, indicating a homogenous transfer of oxygen and nutrients to the cells. The 3D culture system was thus proven to provide supporting conditions for the growth and prolonged maintenance of high-density mesenchymal cells cultures, which can be grown efficiently to an amount sufficient for the purpose of supporting engraftment and successful transplantation.

***3D-ASCs show unique membrane marker characteristics -*** In order to define the difference in the secretion profile of soluble molecules and protein production, effected by the bone environment mimicking 3D culturing procedure, FACs analysis was effected. As is shown in Figure 3a, FACS analysis of cell markers depict that 3D-ASCs display a different marker expression pattern than adherent cells grown in 2D conditions. 2D cultured cells expressed significantly higher levels of positive membrane markers CD90, CD105, CD73 and CD29 membrane markers as compared to 3D cultured cells. For example, CD105 showed a 56 % expression in 3D cultured cells vs. 87 % in 2D cultured cells. ASCs of both 2D and 3D placenta cultures, did not express any hematopoietic membrane markers (Figure 3b).

***3D-ASCs show a unique profile of soluble factors -*** The hematopoietic niche includes supporter cells that produce an abundance of cytokines, chemokines and growth factors. In order to further define the difference between 2D and 3D cultured ASCs, the profile of the four main hematopoietic secreted proteins in the conditioned media of 2D and 3D ASC cultures was effected by ELISA. Figures 4a-c show that cells grown in 3D conditions produced condition media with higher levels of Flt-3 ligand (Figure 4a), IL-60 (Figure 4b), and SCF (Figure 4c), while low levels of IL-6, and close to zero level of Flt-3 ligand and SCF, were detected in the condition media of 2D cultures. Production of Trombopoietin (TPO) was very low and equal in both cultures.

***3D-ASCs show a unique protein profile in mass spectrometry analysis -*** In order to further define the difference between 2D and 3D cultured ASCs, the protein profile of these cells was analyzed by mass spectrometry. Figure 4d shows that 2D and 3D cultured ASCs show a remarkably different protein expression profile. As is shown in Table 1 below, 3D cultured cells show a much higher expression level of H2AF and ALDH X (more than 9 and 12 fold higher, respectively) and a higher level of the proteins EEEF2, RCN2 and CNN1 (ca. 3, 2.5 and 2 fold, respectively). In addition, 3D cultured cells show ca. half the expression levels of the proteins Hnrph1 and CD44 antigen isoform 2 precursor and ca. a third of the expression levels of Papss2 and rpL7a.

**Table 1**

| | | | | |
|---|---|---|---|---|
| protein | Protein level (relative to iTRAQ reporter group) | | | |

| | 3D cultured ASCs | | 2D cultured ASCs | |
|---|---|---|---|---|
| | Av | SD | Av | SD |
| Hnrph1 | 1.434493 | 0.260914 | 0.684687 | 0.197928 |
| H2AF | 0.203687 | 0.288058 | 1.999877 | 0.965915 |
| EEEF2 | 0.253409 | 0.130064 | 0.799276 | 0.243066 |
| RCN2 | 0.54 | 0.25 | 1.34 | 0.26 |
| CD44 antigen isoform 2 precursor | 1.68 | 0.19 | 0.73 | 0.17 |
| CNN1 | 0.77 | 0.15 | 1.55 | 0.17 |
| Papss2 | 1.48352 | 0.314467 | 0.45627 | 0.137353 |
| rpL7a | 1.22 | 0.24 | 0.43 | 0.05 |
| ALDH X | 0.15847 | 0.22411 | 1.986711 | 0.212851 |

***3D-ASCs have the capacity to differentiate into osteoblasts -*** In order to further characterize 3D-ASCs, cells were cultured in an osteoblast differentiating medium for a period of 3 weeks. Thereafter, calcium precipitation was effected. Differentiated cells were shown to produce calcium (depicted in red in Figures 5a-b) whereas control cells maintained a fibroblast like phenotype and demonstrated no mineralization (Figures 5c-d). These results show that placenta derived 3D-ASC have the capacity to differentiate *in vitro* to osteoblasts cells.

### EXAMPLE 2

### Assessment of the Ability of placenta derived 3D-ASC to improve HSC engraftment

3D-ASC support of HSC engraftment was evaluated by the level of human hematopoietic cells (hCD45+) detected in sub lethally irradiated or chemotherapy pretreated immune deficient NOD-SCID mice.

### MATERIALS AND EXPERIMENTAL PROCEDURES

***Isolation of CD34+ Cells -*** Umbilical cord blood samples were taken under sterile conditions during delivery (Bnei Zion Medical Center, Haifa, Israel) and mononuclear cells were fractionated using Lymphoprep (Axis-Shield PoC As, Oslo, Norway) density gradient centrifugation and were cryopreserved. Thawed mononuclear cells were washed and incubated with anti-CD34 antibodies and isolated using midi MACS (Miltenyl Biotech, Bergish Gladbach, Germany). Cells from more than one sample were pooled for achieving the desired amount (50,000-100,000 cells).

***Detection of transplanted cells in irradiated mice -*** Seven week old male and female NOD-SCID mice (NOD-CB17-Prkdcscid/J; Harlan/ Weizmann Inst., Rehovot Israel) were maintained in sterile open system cages, given sterile diets and autoclaved acidic water. The mice were sub lethally irradiated (350 cGy), and thereafter (48 hr post irradiation) transplanted with 50,000-100,000 hCD34⁺ cells, with or without additional ASCs (0.5 X 10⁶ - 1 X 10⁶) derived from placenta or adipose tissue (3-7 mice in each group), by intravenous injection to a lateral tail vein. Four to six weeks following transplantation the mice were sacrificed by dislocation and BM was collected by flushing both femurs and tibias with FACS buffer (50 ml PBS, 5 ml FBS, 0.5 ml sodium azid 5 %). Human cells in the mice BM were detected by flow cytometry, and the percentage of the human and murine CD45 hematopoietic cell marker expressing cells in the treated NOD-SCID mice was effected by incubating cells with anti-human CD45-FITC (IQ Products, Groningen, The Netherlands). The lowest threshold for unequivocal human engraftment was designated at 0.5 %.

***Detection of transplanted cells in mice treated with chemotherapy -*** 6.5 week old male NOD-SCID mice (NOD.CB17/JhkiHsd-scid; Harlan, Rehovot Israel), maintained as described hereinabove for irradiated mice, were injected intraperitoneally with Busulfan (25 mg/kg- for 2 consecutive days). Two days following the second Busulfan injection, mice were injected with CD34+ cells alone, or together with 0.5 X 10⁶ ASCs, produced from the placenta. 3.5 weeks following transplantation, mice were sacrificed, and the presence of human hematopoietic cells was determined as described hereinabove for irradiated mice.

### RESULTS

***3D-ASC improved ertgraftment of HSC in irradiated mice -*** Human CD34+ hematopoietic cells and 3D-ASC derived from placenta or adipose were co-transplanted in irradiated NOD-SCID mice. Engraftment efficiency was evaluated 4 weeks following co-transplantation, and compared to mice transplanted with HSC alone. As is shown in Table 2 and Figure 6, co-transplantation of 3D-ASC and UCB CD34+ cells resulted in considerably higher engraftment rates and higher levels of human cells in the BM of recipient mice compared to mice treated with UCB CD34+ cells alone.

**Table 2**

| **Transplanted cells** | **Average h-CD45** | **STDEV** |
|---|---|---|
| CD34 | 3.8 | 7.9 |
| CD34+3D-ASC from placenta | 5.1 | 12.2 |
| CD34+3D-ASC from adipose | 8.7 | 9.6 |

***3D-ASC improved engraftmen**t **of HSC in mice treated with chemotherapy-***Human CD34+ hematopoietic cells were co-transplanted with 500,000- 2D-ASC or 3D-ASC derived from placenta, into NOD-SCID mice pretreated with chemotherapy. Engraftment efficiency was evaluated 3.5 weeks following co-transplantation, and compared to mice transplanted with HSC alone. As is shown in Table 3, co-transplantation of ASC and UCB CD34+ cells resulted in higher engraftment levels in the BM of the recipient mice compared to UCB CD34+ cells alone. Moreover, as is shown in Table 3, the average level of engraftment was higher in mice co-transplanted with placenta derived adherent cells grown in the PluriX bioreactor system (3D-ASC) than in the mice co-transplantation with cells from the same donor, grown in the conventional static 2D culture conditions (flask).

**Table 3**

| **Transplanted cells** | **Average h-CD45** | **STDEV** |
|---|---|---|
| CD34 | 0.9 | 1.1 |
| CD34 + conventional 2D cultures from placenta | 3.5 | 0.2 |
| CD34 +3D-ASC from placenta | 6.0 | 7.9 |

FACS analysis results shown in Figures 7a-b demonstrate the advantage of co-transplanting ASC with hHSCs (Figure 7b), and the ability of ASC to improve the recovery of the hematopoietic system following HSC transplantation.

Taken together, these results show that ASCs may serve as supportive cells to improve hematopoietic recovery following HSCs transplantation (autologous or allogenic). The ability of the 3D-ASCs to enhance hematopoietic stem and/or progenitor cell engraftment following HSCs transplantation may result from the 3D-ASC ability to secrete HSC supporting cytokines that may improve the homing, self-renewal and proliferation ability of the transplanted cells, or from the ability of those cells to rebuild the damaged hematopoietic microenvironment needed for the homing and proliferation of the transplantable HSCs

### EXAMPLE 3

### The suppression of lymphocyte response by 2D and 3D cultured ASCs

Adherent stromal cells, and particularly 3D-ASCs, were found to suppress the immune reaction of human cord blood mononuclear cells in an MLR assay

### MATERIALS AND EXPERIMENTAL PROCEDURES

***Mixed lymphocyte reaction (MLR) assay -*** The immunosuppressive and immunoprivileged properties of 2D and 3D derived culturing procedures ASCs produced from the placenta, were effected by the MLR assay, which measures histocompatibility at the HLA locus, as effected by the proliferation rate of incompatible lymphocytes in mixed culturing of responsive (proliferating) and stimulating (unproliferative) cells. Human cord blood (CB) mononuclear cells (2 X 10⁵) were used as responsive cells and were stimulated by being co-cultured with equal amounts (10⁵) of irradiated (3000Rad) human peripheral blood derived Monocytes (PBMC), or with 2D or 3D cultured adherent cells, produced from the placenta, or a combination of adherent cells and PBMCs. Each assay was replicated three times. Cells were co-cultured for 4 days in RPMI 1640 medium (containing 20 % FBS under humidified 5 % CO₂ atmosphere at 37 °C), in a 96-well plate. Plates were pulsed with 1µC ³H-thymidine during the last 18 hr of culturing. Cells were then harvested over fiberglass filter and thymidine uptake was quantified with a scintillation counter.

### RESULTS

Figure 8 shows the immune response of CB cells as represented by the elevated proliferation of these cells when stimulated with PBMCs, which, without being bound by theory, is probably associated with T cell proliferation in response to HLA incompatibility. However, a considerably lower level of immune response was exhibited by these cells when incubated with the adherent cells of the present invention. Moreover, the CB immune response to PBMCs was substantially reduced when co-incubated with these adherent cells. Thus, in a similar manner to MSCs, ASCs were found to have the potential ability to reduce T cell proliferation of donor cells, typical of GvHD. Although both cultures, 2D and 3D, reduced the immune response of the lymphocytes, and in line with the other advantages of 3D-ASCs described hereinabove, the 3D ASCs were more immunosuppressive.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. In addition, citation or identification of any reference in this application shall not be construed as an admission that such reference is available as prior art to the present invention.

In view of the above, it will be appreciated that the description also encompasses the following items:
1. A method of cell expansion, the method comprising culturing adherent cells from placenta or adipose tissue under three-dimensional culturing conditions, which support cell expansion.
2. A method of producing a conditioned medium, the method comprising
   (a) culturing adherent cells from a placenta or adipose tissue in three dimensional culturing conditions which allow cell expansion; and
   (b) collecting a conditioned medium of said expanded adherent cells, thereby producing the conditioned medium.
3. A population of cells generated according to the method of item 1.
4. An isolated population of cells comprising adherent cells of placenta or adipose tissue, wherein said adherent cells secrete a higher level of at least one factor selected from the group consisting of SCF, IL-6, and Flt-3 than that secreted by adherent cells of placenta or adipose tissue grown in a 2D culture.
5. An isolated population of cells comprising adherent cells of placenta or adipose tissue, wherein said adherent cells express a higher level of at least one protein selected from the group consisting of H2A histone family (H2AF), Aldehyde dehydrogenase X (ALDH X), eukaryotic translation elongation factor 2 (EEEF2), reticulocalbin 3, EF-hand calcium binding domain (RCN2) and calponin 1 basic smooth muscle (CNN1) than that expressed by adherent cells of placenta or adipose tissue grown in a 2D culture.
6. An isolated population of cells comprising adherent cells of placenta or adipose tissue, wherein said adherent cells express a lower level of expression of at least one protein selected from the group consisting of heterogeneous nuclear ribonucleoprotein H1 (Hnrph1), CD44 antigen isoform 2 precursor, 3 phosphoadenosine 5 phosphosulfate synthase 2 isoform a (Papss2) and ribosomal protein L7a (rpL7a) than that expressed by adherent cells of placenta or adipose tissue grown in a 2D culture.
7. An isolated population of cells comprising adherent cells of placenta or adipose tissue, wherein said adherent cells are characterized by a higher immunosuppressive activity than that of adherent cells of placenta or adipose tissue grown in a 2D culture.
8. The isolated population of cells of item 7, wherein said immunosuppressive activity comprises reduction in T cell proliferation.
9. A pharmaceutical composition comprising, as an active ingredient, the population of cells generated according to item 1.
10. A pharmaceutical composition comprising, as an active ingredient, the conditioned medium produced according to item 2.
11. A pharmaceutical composition comprising, as an active ingredient, the isolated population of cells of item 4, 5, 6 or 7.
12. A method of treating a condition which may benefit from stromal cell transplantation in a subject in need thereof, the method comprising administering to the subject a therapeutically effective amount of adherent cells of a tissue selected from the group consisting of placenta and adipose tissue, thereby treating the condition which may benefit from stem cell transplantation in the subject.
13. A method of treating a condition which may benefit from stromal cell transplantation in a subject in need thereof, the method comprising administering to the subject a therapeutically effective amount of a conditioned medium of adherent cells derived from a tissue selected from the group consisting of placenta and adipose tissue, thereby treating the condition which may benefit from stem cell transplantation in the subject.
14. A method of reducing an immune response in a subject in need thereof, the method comprising administering to the subject a therapeutically effective amount of the isolated population of cells of item 3, 4, 5, 6 or 7, so as to reduce the immune response in the subject.
15. The method of item 14, wherein the subject is treated with cell therapy.
16. The method of item 12 or 13, further comprising administering stem cells.
17. The method of item 16, wherein said stem cells comprise hematopoietic stem cells.
18. The method of item 16, wherein said stem cells are administered concomitantly with said conditioned medium or adherent cells.
19. The method of item 16, wherein said stem cells are administered following administration of said conditioned medium or adherent cells.
20. The method of items 12 and 13, wherein said adherent cells are obtained from a three dimensional culture.
21. The method of items 12 and 13, wherein said adherent cells are obtained from a two dimensional culture.
22. The method of items 12 and 13, wherein said condition is selected from the group consisting of stem cell deficiency, heart disease, Parkinson's disease, cancer, Alzheimer's disease, stroke, burns, loss of tissue, loss of blood, anemia, autoimmune disorders, diabetes, arthritis, Multiple Sclerosis, graft vs. host disease (GvHD), neurodegenerative disorders, autoimmune encephalomyelitis (EAE), systemic lupus erythematosus (SLE), rheumatoid arthritis, systemic sclerosis, Sjorgen's syndrome, multiple sclerosis (MS), Myasthenia Gravis (MG), Guillain-Barré Syndrome (GBS), Hashimoto's Thyroiditis (HT), Graves's Disease, Insulin dependent Diabetes Melitus (IDDM) and Inflammatory Bowel Disease.
23. The method or cell population of any of item 1, 2, 3 or 20, wherein said three dimensional culture comprises a 3D bioreactor.
24. The method of item 23, wherein said bioreactor is selected from the group consisting of a plug flow bioreactor, a continuous stirred tank bioreactor and a stationary-bed bioreactor.
25. The method or cell population of any of item 1, 2, 3 or 20, wherein said culturing of said cells is effected under a continuous flow of a culture medium.
26. The method or cell population of any of item 1, 2, 3 or 20, wherein said three dimensional culture comprises an adherent material selected from the group consisting of a polyester, a polyalkylene, a polyfluorochloroethylene, a polyvinyl chloride, a polystyrene, a polysulfone, a cellulose acetate, a glass fiber, a ceramic particle, a matrigel, an extracellular matrix component, a collagen, a poly L lactic acid and an inert metal fiber.
27. The method or cell population of any of item 1, 2, 3, or 20, wherein said culturing is effected for at least 3 days.
28. The method or cell population of item 21, wherein said culturing is effected for at least 3 days.
29. The method or cell population of any of item 1, 2, 3 or 20, wherein said culturing is effected until said adherent cells reach at least 60 % confluence.
30. The methods of items 12 and 13, wherein the condition may benefit from the facilitation of hematopoietic stem cell engraftment.
31. The methods, population of cells or medium of item 1, 2, 3, 4, 5, 6, 7 or 20, wherein said adherent cells comprise a positive marker expression array selected from the group consisting of CD73, CD90, CD29 and CD105.
32. The methods, population of cells or medium of item 1, 2, 3, 4, 5, 6, 7 or 20, wherein said adherent cells comprise a negative marker expression array selected from the group consisting of CD45, CD80, HLA-DR, CD11b, CD14, CD19, CD34 and CD79.
33. The methods, population of cells or medium of item 1, 2, 3, or 20, wherein said adherent cells secrete a higher level of at least one factor selected from the group consisting of SCF, Flt-3 and IL-6 higher than that secreted by adherent cells from placenta or adipose tissue grown in a 2D culture.
34. The methods, population of cells or medium of item 1, 2, 3, or 20, wherein said adherent cells express a higher level of at least one protein selected from the group consisting of H2A histone family (H2AF), Aldehyde dehydrogenase X (ALDH X), eukaryotic translation elongation factor 2 (EEEF2), reticulocalbin 3, EF-hand calcium binding domain (RCN2) and calponin 1 basic smooth muscle (CNN1) than that secreted by adherent cells from placenta or adipose tissue grown in a 2D culture.
35. The methods, population of cells or medium of item 1, 2, 3, or 20, wherein said adherent cells express a lower level of expression of at least one protein selected from the group consisting of heterogeneous nuclear ribonucleoprotein H1 (Hnrph1), CD44 antigen isoform 2 precursor, 3 phosphoadenosine 5 phosphosulfate synthase 2 isoform a (Papss2) and ribosomal protein L7a (rpL7a) than that secreted by adherent cells from placenta or adipose tissue grown in a 2D culture.
36. The methods, population of cells or medium of item 1, 2, 3, or 20, wherein said adherent cells or medium are characterized by a higher immunosuppressive activity than that of adherent cells of placenta or adipose tissue grown in a 2D culture.
37. The isolated population of cells of item 36, wherein said immunosuppressive activity comprises reduction in T cell proliferation.
38. The method or cell population of any of item 1, 2, 3, 4, 5, 6, 7, 12, or 13, wherein said cells comprise cells having a stromal stem cell phenotype.
39. The method of item 38, wherein said stromal stem cell phenotype comprises T cell suppression activity.
40. The method of item 38, wherein said stromal stem cell phenotype comprises hematopoietic stem cell support activity.
41. Use of the population of cells of item 3, 4, 5, 6, or 7 for manufacture of a medicament identified for transplantation.

## Claims

1. Adherent stromal cells of a tissue selected from the group consisting of placenta and adipose tissue for use in treating
(a) a condition selected from the group consisting of loss of blood, anemia, autoimmune disorders, arthritis, Multiple Sclerosis, graft vs. host disease (GvHD), autoimmune encephalomyelitis (EAE), systemic lupus erythematosus (SLE), rheumatoid arthritis, systemic sclerosis, Sjorgen's syndrome, Myasthenia Gravis (MG), Guillain-Barre Syndrome (GBS), Hashimoto's Thyroiditis (HT), Graves's Disease, Insulin-dependent Diabetes Mellitus (IDDM) and Inflammatory Bowel Disease; or
(b) a condition that may benefit from the facilitation of hematopoietic stem cell engraftment in a subject,
wherein said adherent stromal cells are obtained from a three dimensional culture.

2. A conditioned medium of adherent stromal cells derived from a tissue selected from the group consisting of placenta and adipose tissue for use in treating a condition selected from the group consisting of autoimmune disorders, arthritis, Multiple Sclerosis, graft vs. host disease (GvHD), autoimmune encephalomyelitis (EAE), systemic lupus erythematosus (SLE), rheumatoid arthritis, systemic sclerosis, Sjorgen's syndrome, Myasthenia Gravis (MG), Guillain-Barre Syndrome (GBS), Hashimoto's Thyroiditis (HT), Graves's Disease, Insulin dependent Diabetes Mellitus (IDDM) and Inflammatory Bowel Disease,
wherein said adherent stromal cells are obtained from a three dimensional culture.

3. The cells or the medium for the use of claims 1 or 2, further comprising administering stem cells.

4. The cells or the medium for the use of claim 3, wherein said stem cells
• comprise hematopoietic stem cells and/or
• are administered concomitantly with said conditioned medium or adherent cells and/or
• are administered following administration of said conditioned medium or adherent cells.

5. The cells or the medium for the use of claim 1, wherein said three dimensional culture comprises a 3D bioreactor.

6. The cells or the medium for the use of claim 5, wherein said bioreactor is selected from the group consisting of a plug flow bioreactor, a continuous stirred tank bioreactor and a stationary-bed bioreactor.

7. The cells or the medium for the use of claim 1, wherein
• said culturing of said cells is effected under a continuous flow of a culture medium and/or
• said three dimensional culture comprises an adherent material selected from the group consisting of a polyester, a polyalkylene, a polyfluorochloroethylene, a polyvinyl chloride, a polystyrene, a polysulfone, a cellulose acetate, a glass fiber, a ceramic particle, a matrigel, an extracellular matrix component, a collagen, a poly L lactic acid and an inert metal fiber and/or
• said culturing is effected for at least 3 days and/or
• said culturing is effected until said adherent cells reach at least 60 % confluence and/or
• said adherent cells comprise a positive marker expression array selected from the group consisting of CD73, CD90, CD29 and CD105 and/or
• said adherent cells comprise a negative marker expression array selected from the group consisting of CD45, CD80, HLA-DR, CDIIb, CD14, CD19, CD34 and CD79 and/or
• said adherent cells secrete a higher level of at least one factor selected from the group consisting of SCF, Flt-3 and IL-6 higher than that secreted by adherent cells from placenta or adipose tissue grown in a 2D culture and/or
• said adherent cells express a higher level of at least one protein selected from the group consisting of H2A histone family (H2AF), Aldehyde dehydrogenase X (ALDH X), eukaryotic translation elongation factor 2 (EEEF2), reticulocalbin 3, EF- hand calcium binding domain (R.CN2) and calponin 1 basic smooth muscle (CNNI) than that secreted by adherent cells from placenta or adipose tissue grown in a 2D culture and/or
• said adherent cells express a lower level of expression of at least one protein selected from the group consisting of heterogeneous nuclear ribonucleoprotein HI (Hnrph1), CD44 antigen isoform 2 precursor, 3 phosphoadenosine 5 phosphosulfate synthase 2 isoform a (Papss2) and ribosomal protein L7a (rpL7a) than that secreted by adherent cells from placenta or adipose tissue grown in a 2D culture.

8. The cells or the medium for the use of claim 1, wherein said adherent cells or medium are **characterized by** a higher immunosuppressive activity than that of adherent cells of placenta or adipose tissue grown in a 2D culture.

9. The cells or the medium for the use of claim 8, wherein said immunosuppressive activity comprises reduction in T cell proliferation.

10. The cells or the medium for the use of claims 1 or 2, wherein said cells comprise cells having a stromal stem cell phenotype.

11. The cells or the medium for the use of claim 10, wherein said stromal stem cell phenotype comprises T cell suppression activity and/or hematopoietic stem cell support activity.

## Patentansprüche

1. Adhärente Stromazellen eines Gewebes, das aus der Gruppe ausgewählt ist, die aus Plazenta- und Fettgewebe besteht, zur Verwendung beim Behandeln
(a) eines Zustands, der aus der Gruppe ausgewählt ist, die aus Blutverlust, Anämie, Auto-immunerkrankungen, Arthritis, multipler Sklerose, Graft-versus-Host-Krankheit (GvHD), autoimmuner Enzephalomyelitis (EAE), systemischem Lupus erythematodes (SLE), rheumatoider Arthritis, systemischer Sklerose, Sjögren-Syndrom, Myasthenia gravis (MG), Guillain-Barré-Syndrom (GBS), Hashimoto-Thyreoiditis (HT), Morbus Basedow, Insulin-abhängigem Diabetes mellitus (IDDM) und entzündlicher Darmkrankheit besteht; oder
(b) eines Zustands, der von der Durchführung einer Transplantation hämatopoietischer Stammzellen in ein Individuum profitieren könnte,
wobei jene adhärenten Stromazellen aus einer dreidimensionalen Kultur erhalten werden.

2. Konditioniertes Medium adhärenter Stromazellen, die aus einem Gewebe stammen, das aus der Gruppe ausgewählt ist, die aus Plazenta- und Fettgewebe besteht, zur Verwendung beim Behandeln eines Zustands, der aus der Gruppe ausgewählt ist, die aus Autoimmunerkrankungen, Arthritis, multipler Sklerose, Graft-versus-Host-Krankheit (GvHD), autoimmuner Enzephalomyelitis (EAE), systemischem Lupus erythematodes (SLE), rheumatoider Arthritis, systemischer Sklerose, Sjögren-Syndrom, Myasthenia gravis (MG), Guillain-Barré-Syndrom (GBS), Hashimoto-Thyreoiditis (HT), Morbus Basedow, Insulin-abhängigem Diabetes mellitus (IDDM) und entzündlicher Darmkrankheit besteht,
wobei jene adhärenten Stromazellen aus einer dreidimensionalen Kultur erhalten werden.

3. Zellen oder Medium zur Verwendung nach Anspruch 1 oder 2, ferner umfassend das Verabreichen von Stammzellen.

4. Zellen oder Medium zur Verwendung nach Anspruch 3, wobei die Stammzellen
• hämatopoietische Stammzellen umfassen und/oder
• gleichzeitig mit dem konditionierten Medium oder den adhärenten Zellen verabreicht werden und/oder
• nach der Verabreichung des konditionierten Mediums oder der adhärenten Zellen verabreicht werden.

5. Zellen oder Medium zur Verwendung nach Anspruch 1, wobei die dreidimensionale Kultur einen 3D-Bioreaktor umfasst.

6. Zellen oder Medium zur Verwendung nach Anspruch 5, wobei der Bioreaktor aus der Gruppe ausgewählt ist, die aus einem Pfropfenstrombioreaktor, einem kontinuierlich gerührten Tank-Bioreaktor und einem Bioreaktor mit stationärem Bett besteht.

7. Zellen oder Medium zur Verwendung nach Anspruch 1, wobei
• das Kultivieren der Zellen unter einem kontinuierlichen Strom eines Kulturmediums durchgeführt wird und/oder
• die dreidimensionale Kultur ein adhärentes Material umfasst, das aus der Gruppe ausgewählt ist, die aus einem Polyester, einem Polyalkylen, einem Polyfluorchlorethylen, einem Polyvinylchlorid, einem Polystyrol, einem Polysulfon, einem Celluloseacetat, einer Glasfaser, einem Keramikpartikel, einem Matrigel, einer Komponente der extrazellulären Matrix, einem Collagen, einer Poly-L-Milchsäure und einer inerten Metallfaser besteht, und/oder
• das Kultivieren für mindestens 3 Tage durchgeführt wird und/oder
• das Kultivieren durchgeführt wird, bis die adhärenten Zellen eine Konfluenz von mindestens 60 % erreichen, und/oder
• die adhärenten Zellen ein Positivmarker-Expressionsarray umfassen, ausgewählt aus der Gruppe bestehend aus CD73, CD90, CD29 und CD105, und/oder
• die adhärenten Zellen ein Negativmarker-Expressionsarray umfassen, ausgewählt aus der Gruppe bestehend aus CD45, CD80, HLA-DR, CDIIb, CD14, CD19, CD34 und CD79, und/oder
• die adhärenten Zellen eine höhere Menge mindestens eines Faktors sezernieren, der aus der Gruppe ausgewählt ist, die aus SCF, Flt-3 und IL-6 besteht, als adhärente Zellen aus Plazenta- oder Fettgewebe, die in einer 2D-Kultur wachsen, und/oder
• die adhärenten Zellen mindestens ein Protein, das aus der Gruppe ausgewählt ist, die aus der H2A-Histonfamilie (H2AF), Aldehyddehydrogenase X (ALDH X), dem eukaryotischen Translationselongationsfaktor 2 (EEEF2), Reticulocalbin 3, der EF-Hand-Calcium-Bindungsdomäne (R.CN2) und basischem Calponin-1 aus Glattmuskel (CNNI) besteht, in höherer Menge exprimieren als adhärente Zellen aus Plazenta- oder Fettgewebe, die in einer 2D-Kultur wachsen, und/oder
• die adhärenten Zellen mindestens ein Protein, das aus der Gruppe ausgewählt ist, die aus dem heterogenen nukleären Ribonukleoprotein HI (Hnrph1 der Isoform-2-Vorstufe des CD44-Antigens, 3-Phosphoadenosin-5-phosphosulfatsynthase-2-Isoform a (Papss2) und dem ribosomalen Protein L7a (rpL7a) besteht, in geringerer Menge exprimieren als adhärente Zellen aus Plazenta- oder Fettgewebe, die in einer 2D-Kultur wachsen.

8. Zellen oder Medium zur Verwendung nach Anspruch 1, wobei die adhärenten Zellen oder das Medium durch eine höhere immunsuppressive Aktivität gekennzeichnet sind als adhärente Zellen aus Plazenta- oder Fettgewebe, die in einer 2D-Kultur wachsen.

9. Zellen oder Medium zur Verwendung nach Anspruch 8, wobei die immunsuppressive Aktivität Reduzierung der T-Zell-Proliferation umfasst.

10. Zellen oder Medium zur Verwendung nach Anspruch 1 oder 2, wobei die Zellen Zellen umfassen, die einen Stromastammzellenphänotyp aufweisen.

11. Zellen oder Medium zur Verwendung nach Anspruch 10, wobei der Stromastammzellenphänotyp T-Zellen-supprimierende Aktivität und/oder hämatopoietische Stammzellenunterstützende Aktivität umfasst.

## Revendications

1. Cellules stromales adhérentes d'un tissu sélectionné dans le groupe constitué de placenta et de tissu adipeux pour son utilisation dans le traitement
(a) d'une affection sélectionnée dans le groupe constitué d'une perte de sang, de l'anémie, de troubles auto-immuns, de l'arthrite, de la sclérose en plaques, de la maladie du greffon contre l'hôte (GvHD), de l'encéphalomyélite auto-immune (EAE), du lupus érythémateux systémique (SLE), de la polyarthrite rhumatoïde, de la sclérose systémique, du syndrome de Sjorgen, de la myasthénie grave (MG), du syndrome de Guillain-Barré (GBS), de la thyroïdite de Hashimoto (HT), de la maladie de Grave, du diabète sucré insulino-dépendant (DID) et d'une affection abdominale inflammatoire ; ou
(b) d'une affection qui peut bénéficier de la facilitation d'une prise de greffe de cellules souches hématopoïétiques chez un sujet,
dans lesquelles lesdites cellules stromales adhérentes sont obtenues d'une culture en trois dimensions.

2. Milieu conditionné de cellules stromales adhérentes dérivées d'un tissu sélectionné dans le groupe constitué de placenta et de tissu adipeux pour son utilisation dans le traitement d'une affection sélectionnée dans le groupe constitué de troubles auto-immuns, de l'arthrite, de la sclérose en plaques, de la maladie du greffon contre l'hôte (GvHD), de l'encéphalomyélite auto-immune (EAE), du lupus érythémateux systémique (SLE), de la polyarthrite rhumatoïde, de la sclérose systémique, du syndrome de Sjorgen, de la myasthénie grave (MG), du syndrome de Guillain-Barré (GBS), de la thyroïdite de Hashimoto (HT), de la maladie de Grave, du diabète sucré insulino-dépendant (DID) et d'une affection abdominale inflammatoire,
dans lequel lesdites cellules stromales adhérentes sont obtenues d'une culture en trois dimensions.

3. Cellules ou milieu pour leur utilisation selon la revendication 1 ou 2, comprenant en outre l'administration de cellules souches.

4. Cellules ou milieu pour leur utilisation selon la revendication 3, dans lesquels lesdites cellules souches
• comprennent des cellules souches hématopoïétiques et/ou
• sont administrées en même temps que ledit milieu conditionnés ou lesdites cellules adhérentes et/ou
• sont administrées après l'administration dudit milieu conditionné ou desdites cellules adhérentes.

5. Cellules ou milieu pour leur utilisation selon la revendication 1, dans lequel ladite culture en trois dimensions comprend un bioréacteur en 3D.

6. Cellules ou milieu pour leur utilisation selon la revendication 5, dans lequel ledit bioréacteur est sélectionné dans le groupe constitué d'un bioréacteur à écoulement piston, d'un bioréacteur à agitation continue et d'un bioréacteur à lit fixe.

7. Cellules ou milieu pour leur utilisation selon la revendication 1, dans lesquels
• ladite culture desdites cellules est effectuée sous un écoulement continu d'un milieu de culture et/ou
• ladite culture en trois dimensions comprend un matériel adhérent sélectionné dans le groupe constitué d'un polyester, d'un polyalkylène, d'un polyfluorochloroéthylène, d'un chlorure de polyvinyle, d'un polystyrène, d'une polysulfone, d'un acétate de cellulose, d'une fibre de verre, d'une particule de céramique, d'un matrigel, d'un composant de la matrice extracellulaire, d'un collagène, d'un acide poly-L-lactique et d'une fibre métallique inerte et/ou
• ladite culture est réalisée pendant au moins 3 jours et/ou
• ladite culture est réalisée jusqu'à ce que lesdites cellules adhérentes atteignent une confluence d'au moins 60 % et/ou
• lesdites cellules adhérentes comprennent un réseau d'expression de marqueur positif sélectionné dans le groupe constitué de CD73, CD90, CD29 et CD105 et/ou
• lesdites cellules adhérentes comprennent un réseau d'expression de marqueur négatif sélectionné dans le groupe constitué de CD45, CD80, HLA-DR, CDIIb, CD14, CD19, CD34 et CD79 et/ou
• lesdites cellules adhérentes sécrètent un niveau d'au moins un facteur sélectionné dans le groupe constitué du SCF, du Flt-3 et de l'IL-6, supérieur à celui sécrété par les cellules adhérentes de placenta ou de tissu adipeux cultivées dans une culture en 2D et/ou
• lesdites cellules adhérentes expriment un niveau d'au moins une protéine sélectionnée dans le groupe constitué de la famille de l'histone H2A (H2AF), de l'aldéhyde déshydrogénase X (ALDH X), du facteur d'élongation de la traduction eucaryote 2 (EEEF2), de la réticulocalbine 3, du domaine EF-hand de liaison au calcium (R.CN2) et la calponine 1 basique des muscles lisses (CNN1) supérieur à celui sécrété par les cellules adhérentes de placenta ou de tissu adipeux cultivées dans une culture en 2D et/ou
• lesdites cellules adhérentes expriment un niveau d'expression d'au moins une protéine sélectionnée dans le groupe constitué de la ribonucléoprotéine nucléaire hétérogène H1 (Hnrph1), du précurseur de l'isoforme 2 de l'antigène CD44, de l'isoforme a de la 3-phosphoadénosine 5-phosphosulfate synthase 2 (Papss2) et d'une protéine ribosomique L7a (rpL7a) inférieur à celui sécrété par les cellules adhérentes de placenta ou de tissu adipeux cultivées dans une culture en 2D.

8. Cellules ou milieu pour leur utilisation selon la revendication 1, dans lesquels lesdites cellules adhérentes ou ledit milieu sont **caractérisés par** une activité d'immunosuppression supérieure à celle de cellules adhérentes de placenta ou de tissu adipeux cultivées dans une culture en 2D.

9. Cellules ou milieu pour leur utilisation selon la revendication 8, dans lesquels ladite activité d'immunosuppression comprend la réduction de la prolifération des cellules T.

10. Cellules ou milieu pour leur utilisation selon la revendication 1 ou 2, dans lesquels lesdites cellules comprennent des cellules ayant un phénotype de cellules souches stromales.

11. Cellules ou milieu pour leur utilisation selon la revendication 10, dans lesquels ledit phénotype de cellules souches stromales comprend une activité de suppression des lymphocytes T et/ou une activité de support des cellules souches hématopoïétiques.
